(19) Europäisches Patentamt
European Patent Office
Office européen des brevets

(11) **EP 1 360 280 B1**

(12) **EUROPEAN PATENT SPECIFICATION**

(45) Date of publication and mention of the grant of the patent:
**28.04.2010 Bulletin 2010/17**

(21) Application number: **02710193.0**

(22) Date of filing: **07.02.2002**

(51) Int Cl.:
*C12N 9/10* (2006.01)

(86) International application number:
**PCT/GB2002/000520**

(87) International publication number:
**WO 2002/068616 (06.09.2002 Gazette 2002/36)**

(54) **TISSUE TRANSGLUTAMINASE**

GEWEBE TRANSGLUTAMINASE

TRANSGLUTAMINASE TISSULAIRE

(84) Designated Contracting States:
**AT BE CH CY DE DK ES FI FR GB GR IE IT LI LU MC NL PT SE TR**

(30) Priority: **07.02.2001 GB 0103024**

(43) Date of publication of application:
**12.11.2003 Bulletin 2003/46**

(73) Proprietor: **RSR LIMITED**
**Pentwyn,**
**Cardiff CF23 8HE (GB)**

(72) Inventors:
• **POWELL, Michael**
**Llandaff,**
**Cardiff CF5 1DN (US)**
• **FURMANIAK, Jadwiga**
**Thornhill,**
**Cardiff CF4 9HZ (GB)**
• **SMITH, Bernard, Rees**
**St. Mellons,**
**Cardiff CF3 9XE (GB)**

(74) Representative: **Turner, Craig Robert et al**
**A.A. Thornton & Co.**
**235 High Holborn**
**London WC1V 7LE (GB)**

(56) References cited:
**WO-A-98/03872**

• **DIETERICH W ET AL: "IDENTIFICATION OF TISSUE TRANSGLUTAMINASE AS THE AUTOANTIGEN OF CELIAC DISEASE" NATURE MEDICINE, NATURE PUBLISHING, CO, US, vol. 3, no. 7, 1 July 1997 (1997-07-01), pages 797-801, XP002052979 ISSN: 1078-8956**
• **NAKACHI K ET AL: "Antibodies to tissue transglutaminase: comparison of ELISA and immunoprecipitation assay in the presence and in the absence of calcium ions." CLINICA CHIMICA ACTA;INTERNATIONAL JOURNAL OF CLINICAL CHEMISTRY. NETHERLANDS FEB 2001, vol. 304, no. 1-2, February 2001 (2001-02), pages 75-84, XP002219884 ISSN: 0009-8981**
• **BIAGI F ET AL: "Tissue transglutaminase antibodies in celiac disease." THE AMERICAN JOURNAL OF GASTROENTEROLOGY. UNITED STATES AUG 1999, vol. 94, no. 8, August 1999 (1999-08), pages 2187-2192, XP009000380 ISSN: 0002-9270**
• **SEISSLER J; WOHLRAB U; WUENSCHE C; SCHERBAUM W A; BOEHM B O: "Autoantibodies from patients with coeliac disease recognize distinct functional domains of the autoantigen tissue transglutaminase." CLINICAL AND EXPERIMENTAL IMMUNOLOGY., vol. 125, 25 August 2001 (2001-08-25), pages 216-221, XP002219885**
• **WONG R C W ET AL: "A comparison of 13 guinea pig and human anti-tissue transglutaminase antibody ELISA kits." JOURNAL OF CLINICAL PATHOLOGY. ENGLAND JUL 2002, vol. 55, no. 7, July 2002 (2002-07), pages 488-494, XP009001148 ISSN: 0021-9746**
• **DATABASE SWISSPROT [Online] 2 February 1991 (1991-02-02) "human tTG" Database accession no. M55153 XP002220815**

- DATABASE SWISSPROT [Online] 4 October 1988 (1988-10-04) "guinea pig tTG" Database accession no. M19646 XP002220816
- DATABASE SWISSPROT [Online] 8 October 1997 (1997-10-08) "bovine tTG" Database accession no. E04251 XP002220817
- DATABASE SWISSPROT [Online] 1 October 1998 (1998-10-01) "mouse tTG" Database accession no. AF076928 XP002220818
- DATABASE SWISSPROT [Online] 2 June 1999 (1999-06-02) "Rat tTG" Database accession no. AF106325 XP002220819

**Description**

The present invention relates to tissue transglutaminase (tTG), and more particularly to DNA coding for and polypeptides containing, the primary structural conformation of one or more epitopes of tTG and to the use thereof in the detection of autoantibodies and/or lymphocytes produced in response to tTG.

tTG belongs to the class of transglutaminases (TGs). The TGs are enzymes catalysing an acyl transfer depending on $Ca^{2+}$, the γ-carboxamide groups of peptide-bonded glutamine residues acting as acyl donors. Primarily, protein-bonded lysine residues function as acyl acceptors, so that the transfer results in an ε-(γ-glutamyl)lysine bond. The substrate specificity of the TGs with respect to the acyl donors is very high (depending on the amino acid sequence), whereas an exceptionally wide spectrum of acceptors is available. The covalent peptide bonds formed are highly stable and protease-resistant, resulting in an increased resistance of the crosslinked proteins to' chemical, enzymatic or physical effects.

There is a widespread occurrence of various TGs in miscellaneous organs, tissues, in plasma and interstitial body fluids, correlating with the occurrence of transglutaminase-modified proteins in blood clots, on cell membranes, in the horny layer of the epidermis, in hair, in nails, and in the extracellular matrix. The described TGs may be distinguished by their physical properties, their location in the body, and their primary structure.

tTG is also referred to as cellular, erythrocyte, endothelial, cytoplasmatic, liver, or type II TG, and is a monomer having a molecular weight of 75-85 kDa. The complete amino acid sequence comprising 687 residues has been derived from cDNA. At the protein level, there is an 84% homology between the human enzyme and the enzyme from mouse macrophages and an 81% homology between the human and guinea pig enzymes.

There are a number of diseases that may be associated with tTG and can include autoimmune diseases and inflammatory diseases. In particular, autoimmune diseases associated with an immune reaction to tTG can be autoimmune diseases arising due to gluten sensitive enteropathy, such as coeliac disease or sprue.

Coeliac disease is a disease of the small intestine mucosa, the first manifestation predominantly occurring during the late infant and toddler ages. If the corresponding clinical picture does not occur before the adult age, it is generally termed non-tropical sprue. Thus, both of these terms describe the same disease, which is hereinafter referred to as coeliac disease.

Coeliac disease is estimated to effect about 1 in 300 individuals in Western countries. The characteristic intolerance of dietary gluten present in wheat, barley, rye and related products results in an inflammatory disease of the upper small intestine in genetically susceptible individuals. Typical symptoms include chronic diarrhoea, abdominal distension and failure to thrive. In some patients symptoms of malabsorption such as anaemia and osteoporosis may manifest. Other symptoms include inflammatory lesions in the small bowel, for example, villous atrophy, hypertrophic crypts and increased number of inter-epithelial lymphocytes.

Provided the disease is diagnosed in time the symptoms can generally be treated by adhering to a gluten-free diet. However malabsorption may give rise to severe disease symptoms if the disease is not diagnosed and treated. Ultimately, there is the increased risk of developing an intestinal lymphoma and also other gastrointestinal neoplasias.

Diagnosis of coeliac disease is currently based on clinical symptoms, histological identification of gluten sensitive enteropathy, and serological tests for observing any response to withdrawal of gluten from an individual's diet. An example of a serological test used for diagnosis and monitoring of coeliac disease is an immunofluorescence test measuring endomysial antibody and measurement of autoantibodies to tTG.

WO98/03872 discloses that tTG is an autoantigen assocaited with coeliac disease and specifically discloses an ELISA based on guinea pig tTG of about 60% purity.

It would be advantageous to be able to non-invasively efficiently and reliably diagnose and monitor autoimmune diseases associated with tTG, such as coeliac disease as referred to above. For example, the above described immunofluorescence test measuring endomysial antibody requires special expertise and equipment and is dependent on subjective assessment of fluorescence patterns. It would, therefore, be advantageous to be able to replace the immunofluorescence test measuring endomysial antibody with a non-invasive sensitive and specific test for measuring autoantibodies to tTG.

It is, therefore, an aim of the present invention to provide an assay system, which alleviates some of the aforementioned problems arising in the diagnosis and monitoring of autoimmune diseases associated with an immune reaction to tTG. It is a further aim of the present invention to provide an assay for measuring autoantibodies or lymphocytes produced in response to tTG, with improved sensitivity and specificity and also provide diagnostic kits for use in the simple and rapid detection of autoantibodies or lymphocytes produced in response to tTG substantially as referred to above. A better understanding of tTG, and epitopes thereof, would be beneficial in achieving the above described aims of the present invention.

More particularly, we have now specifically identified that antigenic determinants (or epitopes) of tTG which interact with autoantibodies and / or lymphocytes produced in response to tTG, and present in sera from patients with coeliac disease, are located in the N-terminal and/or central part of the tTG molecule. In particular, our studies have shown that the N-terminal region (typically amino acids 1 to 89) and/or the central region (typically amino acids 401 to 494 or amino acids 401 to 491) of the tTG molecule are particularly important for interaction with autoantibodies and/or lymphocytes produced in response to tTG and present in sera from coeliac patients.

A DNA sequence encoding:

a polypeptide with which autoantibodies and/or lymphocytes produced in response to tTG can interact (under conditions that allow interaction of tTG with such autoantibodies or lymphocytes), and which comprises part or all of the primary structural conformation (that is a continuous sequence of amino acid residues) of one or more epitopes of tTG with which autoantibodies and/or lymphocytes produced in response to'tTG can interact (under conditions that allow interaction of tTG with such autoantibodies or lymphocytes);

comprises:

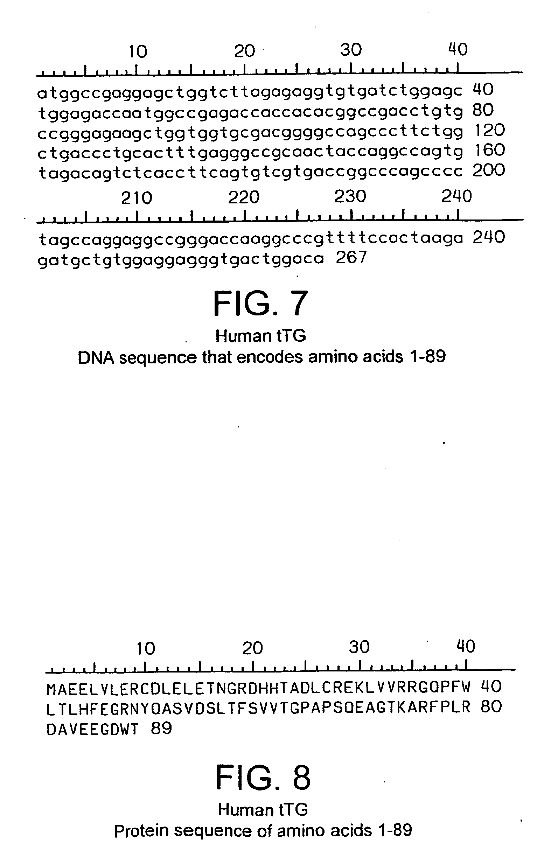

(a) a DNA sequence as depicted in any of Figures 7,9,11,13,15,17,19,21,23 or 25;

(b) a DNA sequence encoding a polypeptide comprising the primary structural conformation of amino acid numbers 1 to 89 of tTG, and / or the primary structural conformation of amino acid numbers 401 to 494 of tTG or the primary structural conformation of amino acid numbers 401 to 491 of tTG, or one or more active fragments of:

amino acid numbers 1 to 89 of tTG, and/or amino acid numbers 401 to 494 of tTG or amino acid numbers 401 to 491 of tTG, with which autoantibodies and/or lymphocytes produced in response to tTG can interact (under conditions that allow interaction of tTG with such autoantibodies or lymphocytes),

with the exception of human, guinea pig, mouse, bovine or rat full length tTG (or more generally full length tTG);

(c) a DNA sequence encoding a polypeptide comprising the primary structural conformation of amino acids as depicted in one or more of Figures 8, 10, 12, 14, 16, 18, 20, 22, 24 and 26, or one or more active fragments of amino acids as depicted in one or more of Figures 8, 10, 12, 14, 16, 18, 20, 22, 24 and 26, with which autoantibodies and/or lymphocytes produced in response to tTG can interact (under conditions that allow interaction of tTG with such autoantibodies or lymphocytes), with the exception of human, guinea pig, mouse, bovine or rat full length tTG (or more generally full length tTG);

(d) a DNA sequence differing from the DNA sequence of (a) in codon sequence due to the degeneracy of the genetic code;

(e) a DNA sequence comprising a fragment or an allelic variation of the DNA sequence of (a); and

(f) a DNA sequence which hybridizes to any of DNA sequences (a), (b), (c), (d) or (e) and encodes a polypeptide with which autoantibodies and/or lymphocytes produced in response to tTG can interact (under conditions that allow interaction of tTG with such autoantibodies or lymphocytes) and which comprises part or all of the primary structural conformation of one or more epitopes of tTG with which autoantibodies and/or lymphocytes produced in response to tTG can interact (under conditions that allow interaction of tTG with such autoantibodies or lymphocytes).

A DNA sequence encoding:

a polypeptide with which autoantibodies and/or lymphocytes produced in response to tTG can interact (under conditions that allow interaction of tTG with such autoantibodies or lymphocytes), and which comprises part or all of the primary structural conformation (that is a continuous sequence of amino acid residues) of one or more epitopes of tTG with which autoantibodies and/or lymphocytes produced in response to tTG can interact (under conditions that allow interaction of tTG with such autoantibodies or lymphocytes);

comprises:

a) a DNA sequence as depicted in any of Figures 7,9,11,13 or 15;

b) a DNA sequence encoding a polypeptide comprising the primary structural conformation of amino acid numbers 1 to 89 of tTG, or one or more active fragments of amino acid numbers 1 to 89 of tTG, with which autoantibodies and/or lymphocytes produced in response to tTG can interact (under conditions that allow interaction of tTG with such autoantibodies or lymphocytes), with the exception of human, guinea pig, mouse, bovine or rat full length tTG (or more generally full length tTG);

(c) a DNA sequence encoding a polypeptide comprising the primary structural conformation of amino acids as depicted in one or more of Figures 8, 10, 12, 14 and 16, or one or more active fragments of amino acids as depicted in one or more of Figures 8, 10, 12, 14 and 16, with which autoantibodies and/or lymphocytes produced in response to tTG can interact (under conditions that allow interaction of tTG with such autoantibodies or lymphocytes), with the exception of human, guinea pig, mouse, bovine or rat full length tTG (or more generally full length tTG);

(d) a DNA sequence differing from the DNA sequence of (a) in codon sequence due to the degeneracy of the genetic code;

(e) a DNA sequence comprising a fragment or an allelic variation of the DNA sequence of (a); and

(f) a DNA sequence which hybridizes to any of DNA sequences (a), (b), (c), (d) or (e) and encodes a polypeptide with which autoantibodies and/or lymphocytes produced in response to tTG can interact (under conditions that allow interaction of tTG with such autoantibodies or lymphocytes) and which comprises part or all of the primary structural conformation of one or more epitopes of tTG with which autoantibodies and/or lymphocytes produced in response to tTG can interact (under conditions that allow interaction of tTG with such autoantibodies or lymphocytes).

A DNA sequence encoding:
a polypeptide with which autoantibodies and/or lymphocytes produced in response to tTG can interact (under conditions that allow interaction of tTG with such autoantibodies or lymphocytes), and which comprises part or all of the primary structural conformation (that is a continuous sequence of amino acid residues) of one or more epitopes of tTG with which autoantibodies and/or lymphocytes produced in response to tTG can interact (under conditions that allow interaction of tTG with such autoantibodies or lymphocytes);
comprises:

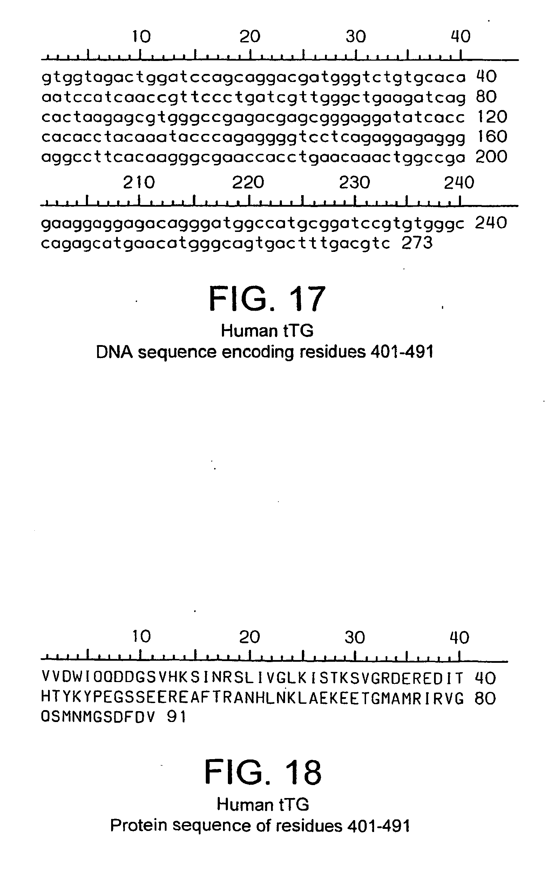

(a) a DNA sequence as depicted in any of Figures 17, 19, 21, 23 or 25;

(b) a DNA sequence encoding a polypeptide comprising the primary structural conformation of amino acid numbers 401 to 494 of tTG or the primary structural conformation of amino acid numbers 401 to 491 of tTG, or one or more active fragments of:
amino acid numbers 401 to 494 of tTG or amino acid numbers 401 to 491 of tTG, with which autoantibodies and/or lymphocytes produced in response to tTG can interact (under conditions that allow interaction of tTG with such autoantibodies or lymphocytes),
with the exception of human, guinea pig, mouse, bovine or rat full length tTG (or more generally full length tTG);

(c) a DNA sequence encoding a polypeptide comprising the primary structural conformation of amino acids as depicted in one or more of Figures 18, 20, 22, 24 and 26, or one or more active fragments of amino acids as depicted in one or more of Figures 18, 20, 22, 24 and 26, with which autoantibodies and/or lymphocytes produced in response to tTG can interact (under conditions that allow interaction of tTG with such autoantibodies or lymphocytes), with the exception of human, guinea pig, mouse, bovine or rat full length tTG (or more generally full length tTG);

(d) a DNA sequence differing from the DNA sequence of (a) in codon sequence due to the degeneracy of the genetic code;

(e) a DNA sequence comprising a fragment or an allelic variation of the DNA sequence of (a); and

(f) a DNA sequence which hybridizes to any of DNA sequences (a), (b), (c), (d) or (e) and encodes a polypeptide with which autoantibodies and/or lymphocytes produced in response to tTG can interact (under conditions that allow interaction of tTG with such autoantibodies or lymphocytes) and which comprises part or all of the primary structural conformation of one or more epitopes of tTG with which autoantibodies and/or lymphocytes produced in response to tTG can interact (under conditions that allow interaction of tTG with such autoantibodies or lymphocytes).

A DNA sequence encoding:
a polypeptide with which autoantibodies produced in response to tTG can interact (under conditions that allow interaction of tTG with such autoantibodies), and which comprises part or all of the primary structural conformation (that is a continuous sequence of amino acid residues) of one or more epitopes of tTG with which autoantibodies produced in response to tTG can interact (under conditions that allow interaction of tTG with such autoantibodies);
comprises:

**EP 1 360 280 B1**

a) a DNA sequence as depicted in any of Figures 7,9,11,13,15,17,19,21,23 or 25;

b) a DNA sequence encoding a polypeptide comprising the primary structural conformation of amino acid numbers 1 to 89 of tTG, and/or the primary structural conformation of amino acid numbers 401 to 494 of tTG or the primary structural conformation of amino acid numbers 401 to 491 of tTG, or one or more active fragments of:
amino acid numbers 1 to 89 of tTG, and/or amino acid numbers 401 to 494 of tTG or amino acid numbers 401 to 491 of tTG, with which autoantibodies produced in response to tTG can interact (under conditions that allow interaction of tTG with such autoantibodies),
with the exception of human, guinea pig, mouse, bovine or rat full length tTG (or more generally full length tTG);

(c) a DNA sequence encoding a polypeptide comprising the primary structural conformation of amino acids as depicted in one or more of Figures 8, 10, 12, 14, 16, 18, 20, 22, 24 and 26, or one or more active fragments of amino acids as depicted in one or more of Figures 8, 10, 12, 14, 16, 18, 20, 22, 24 and 26, with which autoantibodies produced in response to tTG can interact (under conditions that allow interaction of tTG with such autoantibodies), with the exception of human, guinea pig, mouse, bovine or rat full length tTG (or more generally full length tTG);

(d) a DNA sequence differing from the DNA sequence of (a) in codon sequence due to the degeneracy of the genetic code;

e) a DNA sequence comprising a fragment or an allelic variation of the DNA sequence of (a); and

f) a DNA sequence which hybridizes to any of DNA sequences (a), (b), (c), (d) or (e) and encodes a polypeptide with which autoantibodies produced in response to tTG can interact (under conditions that allow interaction of tTG with such autoantibodies) and which comprises part or all of the primary structural conformation of one or more epitopes of tTG with which autoantibodies produced in response to tTG can interact (under conditions that allow interaction of tTG with such autoantibodies).

A DNA sequence encoding:
a polypeptide with which autoantibodies produced in response to tTG can interact (under conditions that allow interaction of tTG with such autoantibodies), and which comprises part or all of the primary structural conformation (that is a continuous sequence of amino acid residues) of one or more epitopes of tTG with which autoantibodies produced in response to tTG can interact (under conditions that allow interaction of tTG with such autoantibodies);
comprises:

a) a DNA sequence as depicted in any of Figures 7,9,11,13 or 15;

b) a DNA sequence encoding a polypeptide comprising the primary structural conformation of amino acid numbers 1 to 89 of tTG, or one or more active fragments of amino acid numbers 1 to 89 of tTG, with which autoantibodies produced in response to tTG can interact (under conditions that allow interaction of tTG with such autoantibodies), with the exception of human, guinea pig, mouse, bovine or rat full length tTG (or more generally full length tTG);

c) a DNA sequence encoding a polypeptide comprising the primary structural conformation of amino acids as depicted in one or more of Figures 8,10,12,14 and 16, or one or more active fragments of amino acids as depicted in one or more of Figures 8,10,12,14 and 16, with which autoantibodies produced in response to tTG can interact (under conditions that allow interaction of tTG with such autoantibodies), with the exception of human, guinea pig, mouse, bovine or rat full length tTG (or more generally full length tTG);

(d) a DNA sequence differing from the DNA sequence of (a) in codon sequence due to the degeneracy of the genetic code;

e) a DNA sequence comprising a fragment or an allelic variation of the DNA sequence of (a); and

f) a DNA sequence which hybridizes to any of DNA sequences (a), (b), (c), (d) or (e) and encodes a polypeptide with which autoantibodies produced in response to tTG can interact (under conditions that allow interaction of tTG with such autoantibodies) and which comprises part or all of the primary structural conformation of one or more epitopes of tTG with which autoantibodies produced in response to tTG can interact (.under conditions that allow interaction of tTG with such autoantibodies).

A DNA sequence encoding:
a polypeptide with which autoantibodies produced in response to tTG can interact (under conditions that allow interaction of tTG with such autoantibodies), and which comprises part or all of the primary structural conformation (that is a continuous sequence of amino acid residues) of one or more epitopes of tTG with which autoantibodies produced in response to tTG can interact (under conditions that allow interaction of tTG with such autoantibodies);
comprises:

a) a DNA sequence as depicted in any of Figures 17,19,21,23 or 25;

b) a DNA sequence encoding a polypeptide comprising the primary structural conformation of amino acid numbers 401 to 494 of tTG or the primary structural conformation of amino acid numbers 401 to 491 of tTG, or one or more active fragments of:
amino acid numbers 401 to 494 of tTG or amino acid numbers 401 to 491 of tTG, with which autoantibodies produced in response to tTG can interact (under conditions that allow interaction of tTG with such autoantibodies), with the exception of human, guinea pig, mouse, bovine or rat full length tTG (or more generally full length tTG);

(c) a DNA sequence encoding a polypeptide comprising the primary structural conformation of amino acids as depicted in one or more of Figures 18, 20, 22, 24 and 26, or one or more active fragments of amino acids as depicted in one or more of Figures 18, 20, 22, 24 and 26, with which autoantibodies produced in response to tTG can interact (under conditions that allow interaction of tTG with such autoantibodies), with the exception of human, guinea pig, mouse, bovine or rat full length tTG (or more generally full length tTG);

(d) a DNA sequence differing from the DNA sequence of (a) in codon sequence due to the degeneracy of the genetic code;

(e) a DNA sequence comprising a fragment or an allelic variation of the DNA sequence of (a); and

(f) a DNA sequence which hybridizes to any of DNA sequences (a), (b), (c), (d) or (e) and encodes a polypeptide with which autoantibodies produced in response to tTG can interact (under conditions that allow interaction of tTG with such autoantibodies) and which comprises part or all of the primary structural conformation of one or more epitopes of tTG with which autoantibodies produced in response to tTG can interact (under conditions that allow interaction of tTG with such autoantibodies).

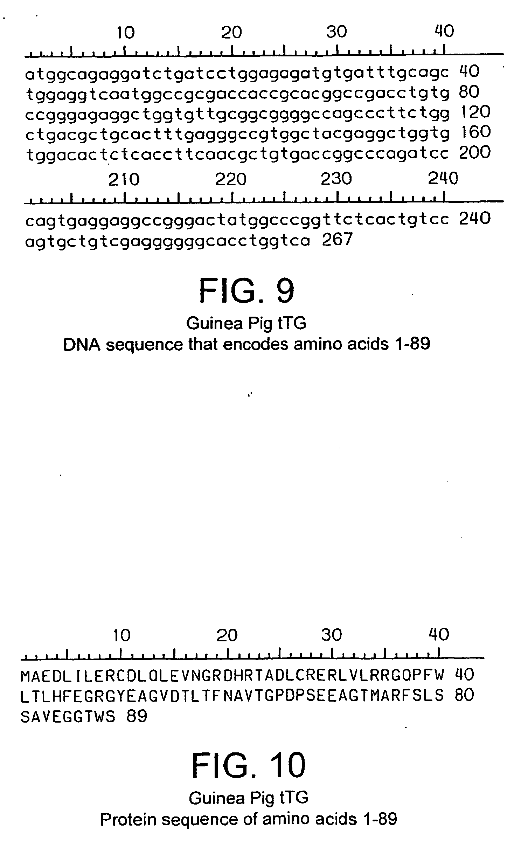

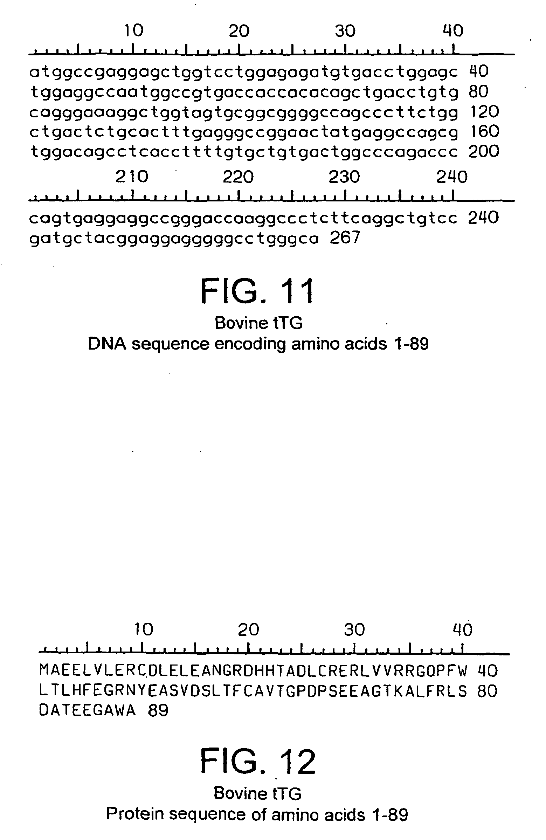

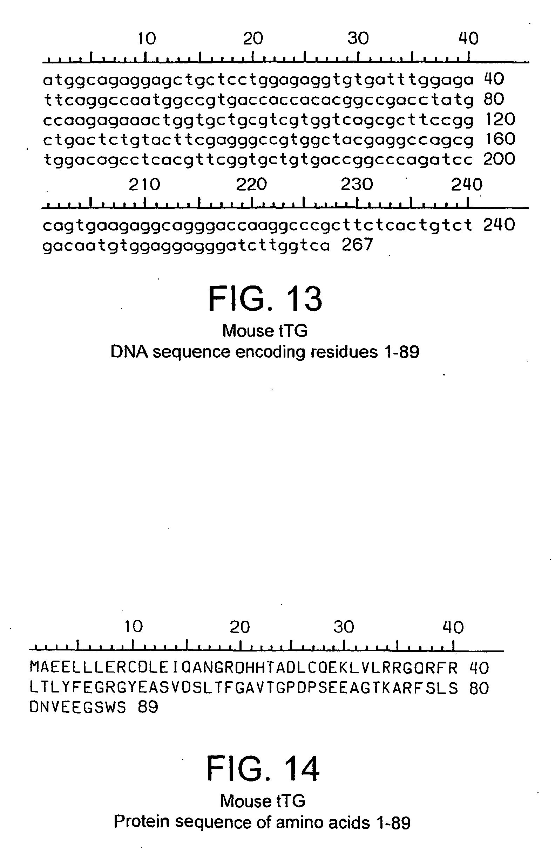

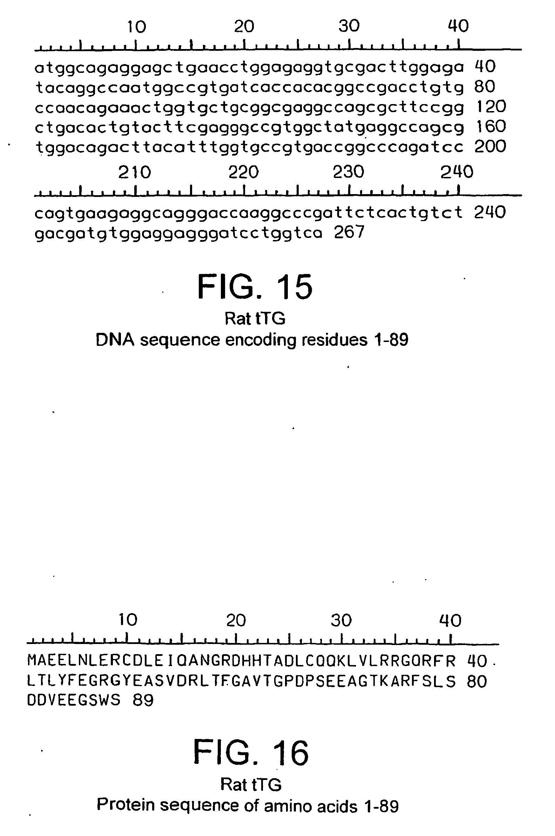

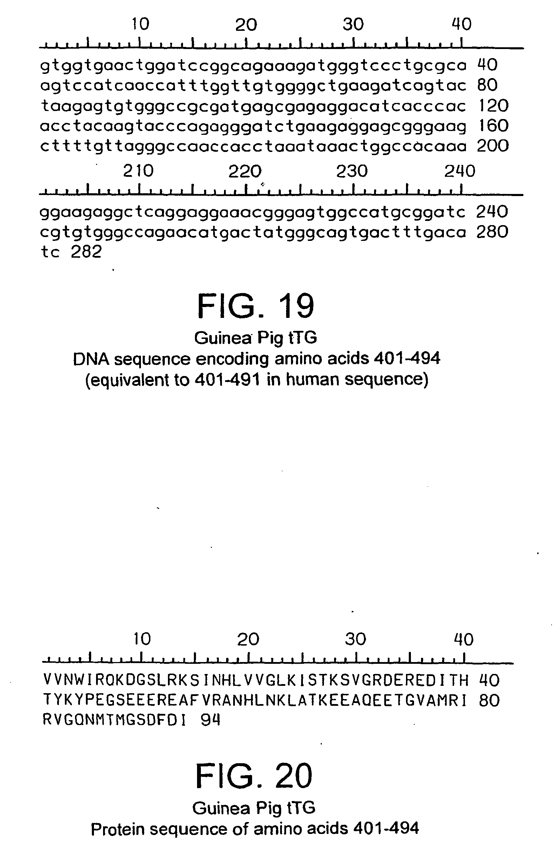

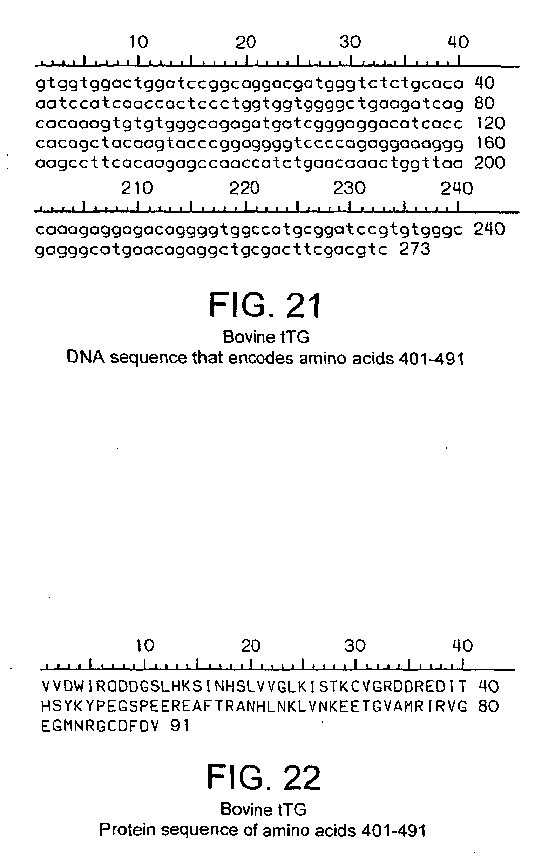

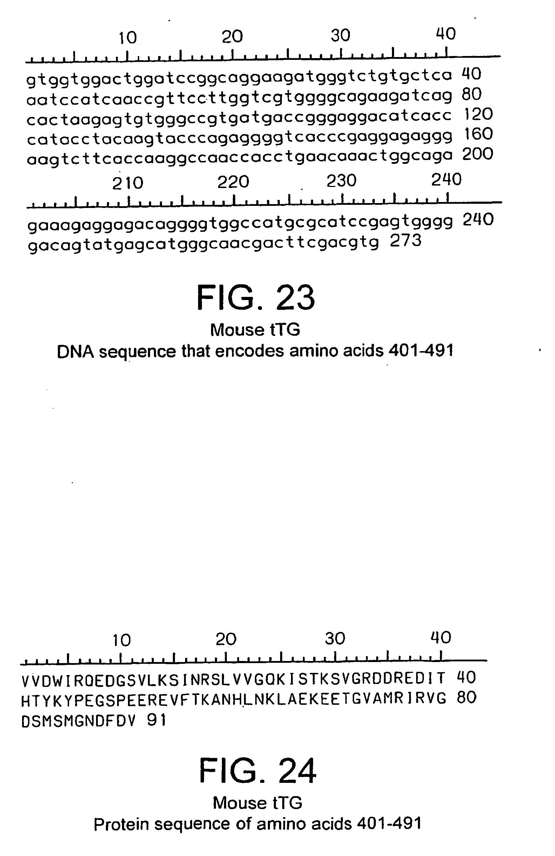

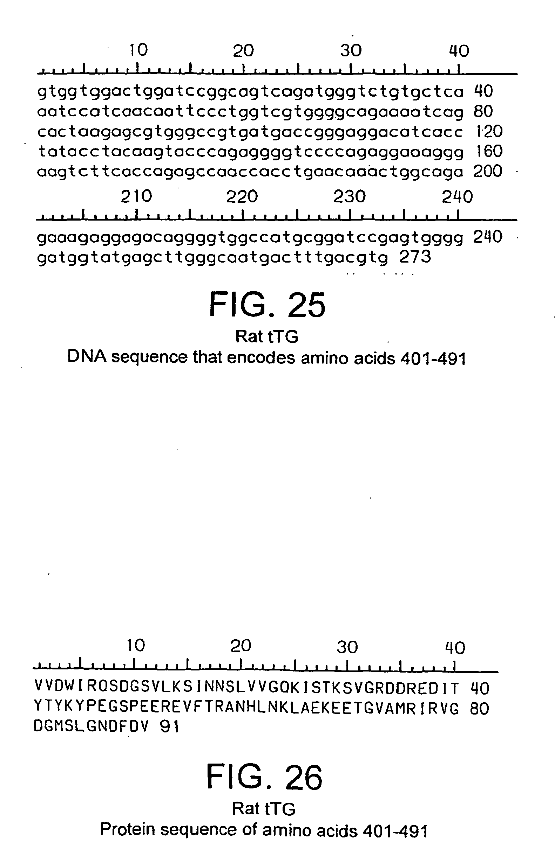

A DNA sequence as described above can be of human origin (as depicted in Figures 7 and 17), of guinea pig origin (as depicted in Figures 9 and 19), of bovine origin (as depicted in Figures 11 and 21), of mouse origin (as depicted in Figures 13 and 23), or of rat origin (as depicted in Figures 15 and 25). In particular, a DNA sequence is as depicted in any of Figures 7,9,11,13,15,17,19,21,23 or 25, or a DNA sequence differing therefrom due to the degeneracy of the genetic code.

More particularly, a DNA sequence can encode part or all of the primary structural conformation of amino acid numbers 1 to 89 of tTG, and/or part or all of the primary structural conformation of amino acid numbers 401 to 494 of tTG or part or all of the primary structural conformation of amino acid numbers 401 to 491 of tTG substantially as hereinafter described in greater detail.

A DNA sequence can be included in a biologically functional plasmid or DNA vector for expressing a polypeptide. A biologically functional plasmid or DNA vector includes a DNA sequence substantially as hereinbefore described. Furthermore, a host cell is transformed or transfected with a DNA sequence substantially as hereinbefore described, or a plasmid or vector substantially as hereinbefore described. A suitable host cell can be any prokaryote or eukaryote cell capable of replicating and expressing a DNA sequence substantially as hereinbefore described and suitable techniques are well known to one of ordinary skill in the art.

Polypeptides substantially as herein described can be expressed in various systems generating recombinant proteins. For example, for expression in *E coli*, cDNA coding for the appropriate polypeptides according to the present invention can be cloned into a vector, such as pMEX8, pGEXcT or pQE81L His or an equivalent. In the case of expression in yeast (for example *Saccharomyces cerevisiae* or *Schizosaccharomyces pombe*), vectors such as pYES2, pESP2 or pYES2/CT or an equivalent, can be employed. AcMNPV (Bac-N Blue) vector or an equivalent can be used for expression in insect cells and pRC/CMV vector or an equivalent can be used for expression in mammalian cells, such as Chinese Hamster Ovary (CHO) cells. A polypeptide can be expressed as a discrete protein, or as a fusion protein linked to, for example, glutathione S transferase (GST) or poly histidine linker. For a discrete protein, affinity column chromatography purification using a mouse monoclonal antibody to the relevant part of tTG coupled to a Sepharose particle can be used. If a tTG fragment is fused to GST, glutathione Sepharose chromatography purification can be used to isolate the fusion protein. Specific proteases can be used to separate GST from tTG peptide and a second round of glutathione Sepharose chromatography can be used to separate GST from a tTG fragment. In the case of peptides linked to poly histidine linker, the purification can be carried out using immobilised metal affinity chromatography.

A polypeptide with which autoantibodies and/or lymphocytes produced in response to tTG can interact (under conditions that allow interaction of tTG with such autoantibodies or lymphocytes) comprises part or all of the primary structural conformation (that is a continuous sequence of amino acid residues) of one or more epitopes of tTG with which autoantibodies and/or lymphocytes produced in response to tTG can interact (under conditions that allow interaction of tTG with such autoantibodies or lymphocytes), which polypeptide comprises the primary structural conformation of amino acid numbers 1 to 89 of tTG, and/or the primary structural conformation of amino acid numbers 401 to 494 of tTG or the primary structural conformation of amino acid numbers 401 to 491 of tTG, or one or more active fragments of:

amino acid numbers 1 to 89 of tTG, and/or amino acid numbers 401 to 494 of tTG or amino acid numbers 401 to 491 of tTG, with which autoantibodies and/or lymphocytes produced in response to tTG can interact (under conditions that allow interaction of tTG with such autoantibodies or lymphocytes);

with the exception of human, guinea pig, mouse, bovine or rat full length tTG (or more generally full length Ttg).

A polypeptide with which autoantibodies and/or lymphocytes produced in response to tTG can interact (under conditions that allow interaction of tTG with such autoantibodies or lymphocytes) comprises part or all of the primary structural conformation (that is a continuous sequence of amino acid residues) of one or more epitopes of tTG with which autoantibodies and/or lymphocytes produced in response to tTG can interact (under conditions that allow interaction of tTG with such autoantibodies or lymphocytes), which polypeptide comprises the primary structural conformation of amino acid numbers 1 to 89 of tTG, or one or more active fragments of amino acid numbers 1 to 89 of tTG, with which autoantibodies and/or lymphocytes produced in response to tTG can interact (under conditions that allow interaction of tTG with such autoantibodies or lymphocytes), with the exception of human, guinea pig, mouse, bovine or rat full length tTG (or more generally full length tTG).

A polypeptide with which autoantibodies and/or lymphocytes produced in response to tTG can interact (under conditions that allow interaction of tTG with such autoantibodies or lymphocytes) comprises part or all of the primary structural conformation (that is a continuous sequence of amino acid residues) of one or more epitopes of tTG with which autoantibodies and/or lymphocytes produced in response to tTG can interact (under conditions that allow interaction of tTG with such autoantibodies or lymphocytes), which polypeptide comprises the primary structural conformation of amino acid numbers 401 to 494 of tTG or the primary structural conformation of amino acid numbers 401 to 491 of tTG, or one or more active fragments of:

amino acid numbers 401 to 494 of tTG or amino acid numbers 401 to 491 of tTG, with which autoantibodies and/or lymphocytes produced in response to tTG can interact (under conditions that allow interaction of tTG with such autoantibodies or lymphocytes);

with the exception of human, guinea pig, mouse, bovine or rat full length tTG (or more generally full length tTG).

A polypeptide with which autoantibodies produced in response to tTG can interact (under conditions that allow interaction of tTG with such autoantibodies) comprises part or all of the primary structural conformation (that is a continuous sequence of amino acid residues) of one or more epitopes of tTG with which autoantibodies produced in response to tTG can interact (under conditions that allow interaction of tTG with such autoantibodies), which polypeptide comprises the primary structural conformation of amino acid numbers 1 to 89 of tTG, and/or the primary structural conformation of amino acid numbers 401 to 494 of tTG or the primary structural conformation of amino acid numbers 401 to 491 of tTG, or one or more active fragments of:

amino acid numbers 1 to 89 of tTG, and/or amino acid numbers 401 to 494 of tTG or amino acid numbers 401 to 491 of tTG, with which autoantibodies produced in response to tTG can interact (under conditions that allow interaction of tTG with such autoantibodies);

with the exception of human, guinea pig, mouse, bovine or rat full length tTG (or more generally full length tTG).

A polypeptide with which autoantibodies produced in response to tTG can interact (under conditions that allow interaction of tTG with such autoantibodies) comprises part or all of the primary structural conformation (that is a continuous sequence of amino acid residues) of one or more epitopes of tTG with which autoantibodies produced in response to tTG can interact (under conditions that allow interaction of tTG with such autoantibodies), which polypeptide comprises the primary structural conformation of amino acid numbers 1 to 89 of tTG, or one or more active fragments of amino acid numbers 1 to 89 of tTG, with which autoantibodies produced in response to tTG can interact (under conditions that allow interaction of tTG with such autoantibodies), with the exception of human, guinea pig, mouse, bovine or rat full length tTG (or more generally full length tTG).

A polypeptide with which autoantibodies produced in response to tTG can interact (under conditions that allow interaction of tTG with such autoantibodies) comprises part or all of the primary structural conformation (that is a continuous sequence of amino acid residues) of one or more epitopes of tTG with which autoantibodies produced in response to tTG can interact (under conditions that allow interaction of tTG with such autoantibodies), which polypeptide comprises the primary structural conformation of amino acid numbers 401 to 494 of tTG or the primary structural conformation of amino acid numbers 401 to 491 of tTG, or one or more active fragments of:

amino acid numbers 401 to 494 of tTG or amino acid numbers 401 to 491 of tTG, with which autoantibodies produced in response to tTG can interact (under conditions that allow interaction of tTG with such autoantibodies);

with the exception of human, guinea pig, mouse, bovine or rat full length tTG (or more generally full length tTG).

A polypeptide with which autoantibodies and/or lymphocytes produced in response to tTG can interact (under conditions that allow interaction of tTG with such autoantibodies or lymphocytes) comprises part or all of the primary structural conformation of one or more epitopes of tTG with which autoantibodies and/or lymphocytes produced in response to tTG can interact (under conditions that allow interaction of tTG with such autoantibodies or lymphocytes), which polypeptide comprises the primary structural conformation of amino acids as depicted in one or more of Figures 8,10, 12,14,16,18,20,22,24 and 26, or one or more active fragments of amino acids as depicted in one or more of Figures 8,10,12,14,16,18,20,22,24 and 26, with which autoantibodies and/or lymphocytes produced in response to tTG can interact (under conditions that allow interaction of tTG with such autoantibodies or lymphocytes), with the exception of human, guinea pig, mouse, bovine or rat full length tTG (or more generally full length tTG).

A polypeptide with which autoantibodies and/or lymphocytes produced in response to tTG can interact (under conditions that allow interaction of tTG with such autoantibodies or lymphocytes) comprises part or all of the primary structural conformation of one or more epitopes of tTG with which autoantibodies and/or lymphocytes produced in response to tTG can interact (under conditions that allow interaction of tTG with such autoantibodies or lymphocytes), which polypeptide comprises the primary structural conformation of amino acids as depicted in one or more of Figures 8,10, 12, 14 and 16, or one or more active fragments of amino acids as depicted in one or more of Figures 8,10,12,14 and 16, with which autoantibodies and/or lymphocytes produced in response to tTG can interact (under conditions that allow interaction of tTG with such autoantibodies or lymphocytes), with the exception of human, guinea pig, mouse, bovine or rat full length tTG (or more generally full length tTG).

A polypeptide with which autoantibodies and/or lymphocytes produced in response to tTG can interact (under conditions that allow interaction of tTG with such autoantibodies or lymphocytes) comprises part or all of the primary structural conformation of one or more epitopes of tTG with which autoantibodies and/or lymphocytes produced in response to tTG can interact (under conditions that allow interaction of tTG with such autoantibodies or lymphocytes), which polypeptide comprises the primary structural conformation of amino acids as depicted in one or more of Figures 18, 20,22,24 and 26, or one or more active fragments of amino acids as depicted in one or more of Figures 18,20,22,24 and 26, with which autoantibodies and/or lymphocytes produced in response to tTG can interact (under conditions that allow interaction of tTG with such autoantibodies or lymphocytes), with the exception of human, guinea pig, mouse, bovine or rat full length tTG (or more generally full length tTG).

A polypeptide with which autoantibodies produced in response to tTG can interact (under conditions that allow interaction of tTG with such autoantibodies) comprises part or all of the primary structural conformation of one or more epitopes of tTG with which autoantibodies produced in response to tTG can interact (under conditions that allow interaction of tTG with such autoantibodies), which polypeptide comprises the primary structural conformation of amino acids as depicted in one or more of Figures 8,10, 12,14,16,18,20,22,24 and 26, or one or more active fragments of amino acids as depicted in one or more of Figures 8,10,12,14,16,18,20,22,24 and 26, with which autoantibodies produced in response to tTG can interact (under conditions that allow interaction of tTG with such autoantibodies), with the exception of human, guinea pig, mouse, bovine or rat full length tTG (or more generally full length tTG).

A polypeptide with which autoantibodies produced in response to tTG can interact (under conditions that allow interaction of tTG with such autoantibodies) comprises part or all of the primary structural conformation of one or more epitopes of tTG with which autoantibodies produced in response to tTG can interact (under conditions that allow interaction of tTG with such autoantibodies), which polypeptide comprises the primary structural conformation of amino acids as depicted in one or more of Figures 8, 10, 12, 14 and 16, or one or more active fragments of amino acids as depicted in one or more of Figures 8,10,12,14 and 16, with which autoantibodies produced in response to tTG can interact (under conditions that allow interaction of tTG with such autoantibodies), with the exception of human, guinea pig, mouse, bovine or rat full length tTG (or more generally full length tTG).

A polypeptide with which autoantibodies produced in response to tTG can interact (under conditions that allow interaction of tTG with such autoantibodies) comprises part or all of the primary structural conformation of one or more epitopes of tTG with which autoantibodies produced in response to tTG can interact (under conditions that allow interaction of tTG with such autoantibodies), which polypeptide comprises the primary structural conformation of amino acids as depicted in one or more of Figures 18, 20,22,24 and 26, or one or more active fragments of amino acids as depicted in one or more of Figures 18,20,22,24 and 26, with which autoantibodies produced in response to tTG can interact (under conditions that allow interaction of tTG with such autoantibodies), with the exception of human, guinea pig, mouse, bovine or rat full length tTG (or more generally full length tTG).

A polypeptide as described above can be of human origin (as depicted in Figures 8 and 18), of guinea pig origin (as depicted in Figures 10 and 20), of bovine origin (as depicted in Figures 12 and 22), of mouse origin (as depicted in Figures 14 and 24), or of rat origin (as depicted in Figures 16 and 26). In particular, the polypeptide is as depicted in any of Figures 8, 10, 12,14,16,18,20,22,24 or 26.

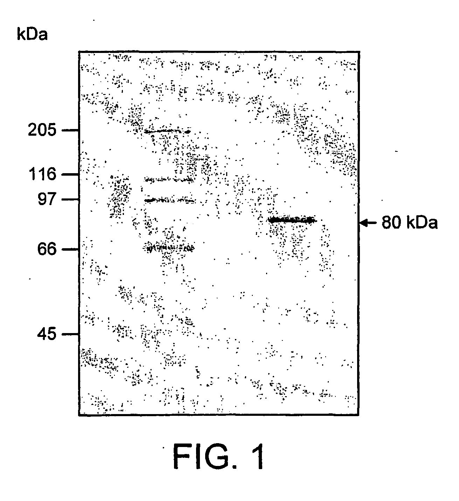

Typically, a polypeptide as described above is a synthetic polypeptide derived by recombinant techniques substantially as herein described and is most suitably derived from recombinant human tTG typically with at least 70% purity, more preferably at least 90% purity and even more preferably at least 95% purity. Purity may be assessed by SDS PAGE analysis and Coomassie Blue staining as hereinafter discussed in connection with Figure 1. The recombinant tTG (typically human) may be purified using affinity chromatography with tTG antibodies such as mouse tTG monoclonal antibody.

A polypeptide as described above is preferably obtained by, or is obtainable by, expression of a DNA sequence according to the present invention substantially as hereinbefore described. A polypeptide obtained by such expression can be advantageous in being free from association with other eukaryotic polypeptides or contaminants which might otherwise be associated with tTG in its natural environment.

A process of preparing a polypeptide substantially as hereinbefore described comprises:

(i) providing a host cell substantially as hereinbefore described;

(ii) growing the host cell; and

(iii) recovering a polypeptide according to the present invention therefrom.

Recovery of a polypeptide can typically employ conventional isolation and purification techniques, such as chromatographic separations or immunological separations, known to one of ordinary skill in the art.

One or more amino acid sequences or fragments thereof structurally distinct from the specific amino acid sequences of a polypeptide described herein (for example by the addition, deletion, substitution or insertion of one or more amino acids) have substantially the same functional binding properties as the specific amino acid sequences of a polypeptide described herein. In other words, amino acid sequences or fragments thereof substantially homologous to specific amino acid sequences of a polypeptide described herein, or representing variants of specific amino acid sequences of a polypeptide described herein, may have substantially the same functional binding properties as the specific amino acid sequences of a polypeptide described herein.

A polypeptide as described above can be a polypeptide with which autoantibodies and/or lymphocytes produced in response to tTG can interact (under conditions that allow interaction of tTG with such autoantibodies or lymphocytes) and comprises part or all of the primary structural conformation (that is a continuous sequence of amino acid residues) of one or more epitopes of tTG with which autoantibodies and/or lymphocytes produced in response to tTG can interact (under conditions that allow interaction of tTG with such autoantibodies or lymphocytes), which polypeptide consists essentially of (or consists of) the primary structural conformation of amino acid numbers 1 to 89 of tTG, and/or the primary structural conformation of amino acid numbers 401 to 494 of tTG or the primary structural conformation of amino acid numbers 401 to 491 of tTG, or more particularly consists essentially of (or consists of) the primary structural conformation of amino acids as depicted in one or more of Figures 8, 10, 12, 14, 16, 18, 20, 22, 24 or 26, substantially as hereinbefore described.

Alternatively, a polypeptide with which autoantibodies and/or lymphocytes produced in response to tTG can interact (under conditions that allow interaction of tTG with such autoantibodies or lymphocytes) comprises part or all of the primary structural conformation (that is a continuous sequence of amino acid residues) of one or more epitopes of tTG with which autoantibodies and/or lymphocytes produced in response to tTG can interact (under conditions that allow interaction of tTG with such autoantibodies or lymphocytes), which polypeptide consists essentially of (or consists of) one or more active fragments of amino acid numbers 1 to 89 of tTG, and/or one or more active fragments of amino acid numbers 401 to 494 of tTG or one or more active fragments of amino acid numbers 401 to 491 of tTG, with which autoantibodies and/or lymphocytes produced in response to tTG can interact (under conditions that allow interaction of tTG with such autoantibodies or lymphocytes), or more particularly consists essentially of (or consists of) the primary structural conformation of one or more active fragments of amino acids as depicted in one or more of Figures 8, 10, 12, 14, 16, 18, 20, 22, 24 or 26, with which autoantibodies and/or lymphocytes produced in response to tTG can interact (under conditions that allow interaction of tTG with such autoantibodies or lymphocytes) substantially as hereinbefore described. For example, a polypeptide consisting essentially of (or consisting of) amino acid numbers 1 to 85 of tTG, amino acid numbers 5 to 89 of tTG, or the like, with which autoantibodies and/or lymphocytes produced in response to tTG can interact (under conditions that allow interaction of tTG with such autoantibodies or lymphocytes).

A polypeptide (in particular a synthetic polypeptide, such as a scaffold type polypeptide typically providing amino acids corresponding to the amino acids of tTG as described herein in a form specifically adapted for use in an assay method or kit according to the present invention substantially as hereinafter described in greater detail) with which autoantibodies and/or lymphocytes produced in response to tTG can interact (under conditions that allow interaction of tTG with such autoantibodies or lymphocytes) includes part or all of the primary structural conformation of one or more epitopes of tTG with which autoantibodies and/or lymphocytes produced in response to tTG can interact (under conditions that allow interaction of tTG with such autoantibodies or lymphocytes), which polypeptide includes amino acids comprising the primary structural conformation of amino acid numbers 1 to 89 of tTG, and/or the primary structural conformation of amino acid numbers 401 to 494 of tTG or the primary structural conformation of amino acid numbers 401 to 491 of tTG, or one or more active fragments of amino acid numbers 1 to 89 of tTG, and/or one or more active fragments of amino acid numbers 401 to 494 of tTG or one or more active fragments of amino acid numbers 401 to 491 of tTG, with which autoantibodies and/or lymphocytes produced in response to tTG can interact (under conditions that allow interaction of tTG with such autoantibodies or lymphocytes); or more particularly which includes amino acids comprising the primary structural conformation of amino acids as depicted in one or more of Figures 8, 10, 12, 14, 16, 18, 20, 22, 24 or 26, or one or more active fragments of amino acids as depicted in one or more of Figures 8, 10, 12, 14, 16, 18, 20, 22, 24 or 26, with which autoantibodies and/or lymphocytes produced in response to tTG can interact (under conditions that allow interaction of tTG with such autoantibodies or lymphocytes), with the exception of human, guinea pig, mouse, bovine or rat full length tTG (or more generally full length tTG).

In a first aspect, the present invention provides a method of screening for autoantibodies to tTG in a sample of body fluid obtained from a subject (in particular a human) suspected of suffering from, susceptible to, having or recovering from autoimmune disease associated with an immune reaction to tTG, said method comprising:

(a) contacting said sample with (i) either a polypeptide which consists of amino acid numbers 1 to 89 of tTG or full length tTG, and (ii) at least one competitor antibody or antibody fragment which binds to amino acid numbers 1 to 89 of tTG, and is capable of competing with autoantibodies to tTG in the interaction thereof with a polypeptide or amino acid sequence which consists of amino acid numbers 1 to 89 of tTG, under conditions that allow interaction of tTG with autoantibodies to tTG, so as to permit either said polypeptide or said full length tTG depending on which is used according to (i) to interact with either autoantibodies to tTG present in said sample or said competitor antibody or antibody fragment; and

(b) monitoring the interaction of said polypeptide or said full length tTG with said autoantibodies present in said sample, thereby providing an indication of the presence of said autoantibodies to tTG in said sample.

Substantially as described above, a method according to the present invention is suitable for screening for autoantibodies to tTG in a sample of body fluid obtained from a subject.

A method according to the present invention may typically employ a control, such as a sample of body fluid from a normal subject, in other words a subject known to be without autoimmune disease associated with an immune reaction to tTG.

Typically, in a method employing non-competitive techniques (which method is not provided by the present invention), monitoring of the degree of interaction of (i) autoantibodies to tTG present in the sample and (ii) a polypeptide substantially as hereinbefore described, can comprise providing labelling means either to a polypeptide according to the present invention substantially as hereinbefore described, or to a binding partner for autoantibodies to tTG, either of which technique would enable monitoring of the above described interaction. For example, such a method may comprise directly or indirectly labelling a polypeptide substantially as hereinbefore described; contacting the thus labelled polypeptide with a sample of body fluid being screened for tTG autoantibodies so as to provide a mixture thereof; and adding to the mixture a binding partner for autoantibodies to tTG (such as an anti-IgG reagent) present in the sample of body fluid, so as to cause precipitation of any complexes of labelled polypeptide and tTG autoantibodies present in the mixture. Alternatively, it may be preferred that such a method further comprises adding a labelled binding partner for tTG autoantibodies (such as a labelled anti IgG reagent, for example protein A or anti-human IgG, or labelled full length tTG or an epitope thereof) to a mixture obtained by contacting (i) a polypeptide substantially as hereinbefore described immobilised to a support and (ii) a sample of body fluid being screened for autoantibodies to tTG.

The method of screening for autoantibodies to tTG in the sample of body fluid according to the present invention utilises the principles employed in known competitive assays. The method according to the present invention employs at least one competitor antibody or antibody fragment capable of competing with autoantibodies to tTG in the interaction thereof with a polypeptide substantially as hereinbefore described.

The competitor employed in the competitive assay method according to the present invention comprises one or more antibodies or fragments thereof, which may be natural or partly or wholly synthetically produced. Preferably a competitor as employed in the present invention comprises a monoclonal, recombinant or polyclonal antibody (especially a monoclonal antibody), capable of competing with tTG autoantibodies in the interaction thereof with a polypeptide substantially as hereinbefore described.

The competitive assay method according to the present invention therefore comprises providing at least one competitor antibody or antibody fragment, such as a monoclonal or polyclonal antibody, whereby the polypeptide substantially as hereinbefore described or full length tTG can interact with either the competitor, such as a monoclonal or polyclonal antibody, or autoantibodies to tTG present in said sample.

Typically monitoring in a competitive assay method according to the present invention comprises comparing:

(i) interaction of a polypeptide substantially as hereinbefore described or full length tTG and one or more competitor antibody or antibody fragment (typically a monoclonal or polyclonal antibody), in the absence of said sample of body fluid being screened (that is a suspected disease sample), optionally in the presence of a sample of body fluid from a normal subject, typically a subject known to be without autoimmune disease associated with an immune reaction to tTG ; with

(ii) interaction of a polypeptide substantially as hereinbefore described or full length tTG and one or more competitor antibodies or antibody fragments (typically a monoclonal or polyclonal antibody), in the presence of said sample of body fluid being screened.

Typically, the comparison involves observing a decrease in interaction of the polypeptide or full length tTG and the competitor in (ii) compared to (i) so as to provide an indication of the presence of autoantibodies to tTG in said sample. Typically, the decrease in interaction can be observed by directly or indirectly labelling the competitor and monitoring any change in the interaction of the thus labelled competitor with the polypeptide substantially as hereinbefore described, or full length tTG, in the absence and in the presence of a sample of body fluid being screened for autoantibodies to tTG. Suitably the polypeptide or full length tTG may be immobilised to facilitate the above mentioned monitoring.

The full length tTG can typically be of human, guinea pig, bovine, mouse or rat origin, preferably human, and more preferably human recombinantly obtained full length tTG. A competitor antibody or antibody fragment for use in such an assay typically comprises a monoclonal or polyclonal antibody (preferably monoclonal) substantially as hereinbefore described.

Suitably a detectable label that can be employed in a method according to the present invention can be selected from the group consisting of enzymic labels, isotopic labels, chemiluminescent labels, fluorescent labels, dyes and the like.

In the case where an isotopic label (such as 1251, 14C, 3H or 35S) is employed, monitoring may therefore comprise measuring radioactivity dependent on binding of a polypeptide according to the present invention substantially as hereinbefore described. Radioactivity is generally measured using a gamma counter, or liquid scintillation counter.

According to a further aspect of the present invention, there is provided a kit for screening for autoantibodies to tTG in a sample of body fluid obtained from a subject (in particular a human) suspected of suffering from, susceptible to, having or recovering from autoimmune disease associated with an immune reaction to tTG, said kit comprising:

(a) either a polypeptide which consists of amino acids 1 to 89 of tTG or full length tTG;
(b) at least one competitor antibody or antibody fragment which binds to amino acid numbers 1 to 89 of tTG , and is capable of competing with autoantibodies to tTG in the interaction thereof with a polypeptide or amino acid sequence which consists of amino acids 1 to 89 of tTG;
(c) means for contacting (i) said sample of body fluid obtained from said subject, (ii) either said polypeptide or said full length tTG, depending on which is used according to (a) and (iii) said competitor antibody or fragment under conditions that allow interaction of tTG with autoantibodies to tTG, so as to permit said polypeptide or full length tTG to interact with either autoantibodies to tTG present in sample, or said competitor; and
(d) means for monitoring the interaction of either said polypeptide or said full length tTG with said autoantibodies present in said sample, thereby providing an indication of the presence of said autoantibodies to tTG in said sample.

Substantially as described above, a kit according to the present invention is suitable for screening for autoantibodies produced in response to tTG in a sample of body fluid obtained from a subject. A kit according to the present invention can, however, be particularly adapted for use in screening for autoantibodies produced in response to tTG in a sample of body fluid obtained from a subject substantially as hereinafter described in greater detail.

A kit according to the present invention may typically further comprise control means, such as means for providing a sample of body fluid from a normal subject, in other words a subject known to be without autoimmune disease associated with an immune reaction to tTG.

A kit for screening for autoantibodies to tTG according to the present invention may comprise means for directly monitoring interaction of (i) autoantibodies to tTG present in the sample of body fluid from the subject and (ii) a polypeptide consisting of amino acid numbers 1 to 89 of tTG or full length tTG.

Typically, in a kit comprising non-competitive assay means, means are provided for monitoring the degree of interaction of (i) autoantibodies to tTG present in the sample and (ii) a polypeptide substantially as hereinbefore described, and can comprise labelling means provided either to a polypeptide substantially as hereinbefore described, or to a binding partner for autoantibodies to tTG, either of which would enable monitoring of the above described interaction. For example, a kit may comprise means for directly or indirectly labelling a polypeptide substantially as hereinbefore described; means for contacting the thus labelled polypeptide with a sample of body fluid being screened for tTG autoantibodies so as to provide a mixture thereof; a binding partner for autoantibodies to tTG (such as an anti-IgG reagent) present in the sample of body fluid; and means for adding the binding partner to the mixture so as to cause precipitation of any complexes of labelled polypeptide and tTG autoantibodies present in the mixture. Alternatively, it may be preferred that a kit further comprises a labelled binding partner for tTG autoantibodies (such as a labelled anti-IgG reagent, for example protein A or anti-human IgG, or labelled full length tTG or an epitope thereof) and means for adding the labelled binding partner to a mixture obtained by contacting (i) a polypeptide substantially as hereinbefore described immobilised to a support and (ii) a sample of body fluid being screened for autoantibodies to tTG.

However, the kit for screening for autoantibodies to tTG in the sample of body fluid according to the present invention comprises competitive assay means. Therefore, the kit according to the present invention further comprises at least one competitor antibody or antibody fragment capable of competing with autoantibodies to tTG in the interaction thereof with a polypeptide consisting of amino acid numbers 1 to 89 or tTG, or full length tTG.

The competitor as employed in the competitive assay kit according to the present invention comprises one or more antibodies or antibody fragments, which may be natural or partly or wholly synthetically produced. Preferably, however, a competitor as employed in the present invention comprises a monoclonal or polyclonal antibody (especially a monoclonal antibody), capable of competing with tTG autoantibodies in the interaction thereof with a polypeptide consisting of amino acid numbers 1 to 89 of tTG.

The kit according to the present invention comprises at least one competitor antibody or antibody fragment, such as a monoclonal or polyclonal antibody, whereby a polypeptide consisting of amino acid numbers 1 to 89 of tTG can interact with either a competitor antibody or antibody fragment, such as a monoclonal or polyclonal antibody, or autoantibodies to tTG present in a sample of body fluid being screened.

Typically monitoring means in a competitive assay kit according to the present invention comprise means for comparing:

(i) interaction of a polypeptide consisting of amino acid numbers 1 to 89 of tTG and one or more competitor antibodies or antibody fragments (typically a monoclonal or polyclonal antibody), in the absence of said sample of body fluid being screened (that is a suspected disease sample), optionally in the presence of a sample of body fluid from a normal subject, typically a subject known to be without autoimmune disease associated with an immune reaction to tTG; with

(ii) interaction of a polypeptide consisting of amino acid numbers 1 to 89 of tTG and one or more competitor antibodies or antibody fragments substantially as hereinbefore described (typically a monoclonal or polyclonal antibody), in the presence of said sample of body fluid being screened.

Typically, the comparison involves observing a decrease in interaction of the polypeptide or full length tTG and the competitor antibody or antibody fragment in (ii) compared to (i) so as to provide an indication of the presence of autoantibodies to tTG in said sample. Typically, the decrease in interaction can be observed by directly or indirectly labelling the competitor and monitoring any change in the interaction of the thus labelled competitor with the polypeptide or full length tTG in the absence and in the presence of a sample of body fluid being screened for autoantibodies to tTG. Suitably the polypeptide or full length tTG may be immobilised to facilitate the above mentioned monitoring.

In one embodiment, there is therefore provided by the present invention a kit for screening for autoantibodies to tTG in a sample of body fluid obtained from a subject (in particular a human) suspected of suffering from, susceptible to, having or recovering from autoimmune disease associated with an immune reaction to tTG, said kit comprising:

(a) full length tTG (typically recombinantly obtained full length tTG);

(b) at least one competitor antibody or antibody fragment capable of competing with autoantibodies to tTG in the interaction thereof with a polypeptide which consists of amino acid numbers to 89 of tTG,

(c) means for contacting said sample of body fluid from said subject, said full length tTG and said competitor antibody or antibody fragment (under conditions that allow interaction of tTG with autoantibodies to tTG), so as to permit said full length tTG to interact with either autoantibodies to tTG present in said sample, or said competitor antibody of antibody fragment; and

(d) means for monitoring the interaction of said full length tTG with said autoantibodies present in said sample, thereby providing an indication of the presence of said autoantibodies to tTG in said sample.

The full length tTGcan typically be of human, guinea pig, bovine, mouse or rat origin, preferably human, and more preferably human recombinantly obtained full length tTG. A competitor antibody or antibody fragment for use in such an assay kit typically comprises a monoclonal or polyclonal antibody (preferably monoclonal) substantially as hereinbefore described.

Suitably a detectable label that can be employed in a kit according to the present invention can be selected from the group consisting of enzymic labels, isotopic labels, chemiluminescent labels, fluorescent labels, dyes and the like.

In the case where an isotopic label (such as $^{125}I$, $^{14}C$, $^{3}H$ or $^{35}S$) is employed, monitoring means may therefore comprise means for measuring radioactivity dependent on binding of a polypeptide according to the present invention substantially as hereinbefore described. Radioactivity is generally measured using a gamma counter, or liquid scintillation counter.

In the case of a kit for screening for lymphocytes, it is generally preferred that means are provided for initially isolating lymphocytes from a sample of body fluid from a subject, using techniques well known to one of ordinary skill in the art, and means are also provided for contacting a tTG polypeptide with such isolated lymphocytes so as to stimulate proliferation of the latter by the former. Means (again known to one of ordinary skill in the art) for monitoring the effect of interaction of a polypeptide according to the present invention and such proliferating lymphocytes, are also provided in such a kit according to the present invention.

An antibody produced in response to one or more epitope regions of tTG, which epitope region comprises part or all of-the primary structural conformation of amino acids 1 to 89 of tTG, or part or all of the primary structural conformation of amino acids 401 to 494 or 401 to 491 of tTG, or one or more fragments of an antibody (such as Fab fragments) produced in response to one or more epitope regions of tTG, which epitope region comprises part or all of the primary structural conformation of amino acids 1 to 89 of tTG, or part or all of the primary structural conformation of amino acids 401 to 494 or 401 to 491 of tTG.

An antibody or antibody fragment as described above thereof can interact with a polypeptide substantially as hereinbefore described and preferably is obtainable by, or is obtained by, techniques described in the Examples. Suitably, such an antibody can be monoclonal (preferred), recombinant or polyclonal. Typically an antibody, such as a monoclonal antibody is in substantially purified form.

More specifically, a monoclonal antibody can comprise a monoclonal antibody Mab A3-6B5, or a monoclonal antibody Mab A5-4E6, or one or more active fragments thereof (such as Fab fragments), as described in the Examples.

A host cell contains an antibody substantially as described above and also a hybridoma is capable of secreting a monoclonal antibody substantially as hereinbefore described.

An antibody substantially as hereinbefore described, or one or more fragments of an antibody (such as Fab fragments) substantially as hereinbefore described, may be used in diagnosis or therapy, in particular diagnosis or therapy of autoimmune disease associated with an immune reaction to tTG substantially as hereinbefore described. Particularly the autoimmune disease can be coeliac disease (although other autoimmune diseases substantially as hereinafter described in greater detail can be diagnosed or treated by such an antibody). An antibody substantially as hereinbefore described may also be used in the manufacture of a medicament for the treatment of coeliac disease.

A polypeptide substantially as hereinbefore described, may be used in diagnosis or therapy, in particular diagnosis or therapy of autoimmune disease associated with an immune reaction to tTG substantially as hereinbefore described.

In particular, autoimmune disease associated with an immune reaction to tTG as described herein comprises autoimmune disease arising due to gluten sensitive enteropathy in a subject, in particular a human subject, such as coeliac disease. Other autoimmune diseases associated with an immune reaction to tTG that can be diagnosed or treated include any of the following-dermatitis herpetiformis, Addison's disease, AI (A1- autoimmune) haemolytic anaemia, A1 thrombocytopenic purpura, AI thyroid diseases, AI hyperthyroidism (Graves' disease), AI hypothyroidism (including Hashimoto's thyroiditis), pernicious anaemia, myasthenia gravis, primary biliary cirrhosis, rheumatoid arthritis, Sjogren's syndrome, SLE, type 1 (insulin-dependent) diabetes mellitus, diabetes mellitus of other types, Wegener granulomatosis, and vitiligo.

Substantially as herein described autoimmune disease associated with an immune reaction to tTG can be screened for or diagnosed in a subject suspected of suffering from, susceptible to, having or recovering from autoimmune disease associated with an immune reaction to tTG. The patient group that can be classed as susceptible to an autoimmune disease associated with an immune reaction to tTG can typically include relatives of subjects previously diagnosed as having an autoimmune disease associated with an immune reaction to tTG, or subjects previously diagnosed with a primary or first autoimmune disease associated with an immune reaction to tTG and as such could be considered to be at risk of or prone to be suffering from a secondary or second autoimmune disease associated with an immune reaction to tTG. For example, a subject known to be suffering from type 1 diabetes mellitus could be considered to be susceptible to suffering from coeliac disease.

It will be appreciated from the foregoing description that the present invention provides assay methods and kits for detecting autoantibodies produced in response to tTG in a sample of body fluid substantially as hereinbefore described. The detection of such autoantibodies and/or lymphocytes produced in response to tTG in the sample of body fluid (or at least the level of such autoantibodies and/or lymphocytes in the sample) is indicative of the presence of autoimmune disease associated with an immune reaction to tTG in the subject from which the sample was obtained and can, therefore, enable the diagnosis of the likely onset or presence of autoimmune disease associated with an immune reaction to tTG.

Therefore, a method of diagnosing the likely onset or presence of autoimmune disease associated with an immune reaction to tTG in a subject (in particular a human) suspected of suffering from, susceptible to, having or recovering from, autoimmune disease associated with an immune reaction to tTG, comprises detecting autoantibodies or lymphocytes produced in response to tTG in a sample of body fluid from the subject substantially as hereinbefore described, whereby the detected autoantibodies and/or lymphocytes can provide a diagnosis of the likely onset or presence of autoimmune disease associated with an immune reaction to tTG in the subject.

A method of delaying or preventing the onset of autoimmune disease associated with an immune reaction to tTG in an animal subject (in particular a human subject) suspected of suffering from, susceptible to or recovering from autoimmune disease associated with an immune reaction to tTG, comprises initially detecting autoantibodies or lymphocytes indicative of the onset or presence of autoimmune disease associated with an immune reaction to tTG in a sample of body fluid obtained from the subject substantially as hereinbefore described, thereby providing a diagnosis of the likely onset of autoimmune disease associated with an immune reaction to tTG in the subject, and thereafter therapeutically treating the subject so as to delay the onset and/or prevent autoimmune disease associated with an immune reaction to tTG. Typically, such therapeutic treatment can, in the case of gluten sensitive enteropathy giving rise to coeliac disease, comprise compliance with a gluten-free or substantially gluten free diet.

A method of monitoring dietary compliance by a subject (in particular a human subject) having, suspected of suffering from, susceptible to or recovering from gluten sensitive enteropathy, comprises screening for autoantibodies or lymphocytes produced in response to tTG in a sample of body fluid obtained from the subject substantially as hereinbefore described, whereby the detection of such autoantibodies and/or lymphocytes produced in response to tTG in the sample of body fluid (or at least the level of such autoantibodies and/or lymphocytes in the sample) provides an indication as to the dietary compliance of the subject to a gluten-free or substantially gluten free diet.

A polypeptide substantially as hereinbefore described is particularly suitable for use in the therapeutic treatment of autoimmune disease associated with an immune reaction to tTG. For example, tolerance can be achieved by administering a polypeptide according to the present invention substantially as hereinbefore described to a subject (in particular a human subject) suspected of suffering from, susceptible to, having or recovering from autoimmune disease associated with an immune reaction to tTG.

A pharmaceutical composition comprises a polypeptide substantially as hereinbefore described, together with a pharmaceutically acceptable carrier, diluent or excipient therefor, wherein the polypeptide can interact with autoantibodies and/or lymphocytes produced in response to tTG.

A polypeptide substantially as hereinbefore described may be used in the manufacture of a medicament for the treatment of coeliac disease.

Compositions or medicaments should contain a therapeutic or prophylactic amount of at least one polypeptide as hereinbefore described in a pharmaceutically-acceptable carrier. The pharmaceutical carrier can be any compatible, non-toxic substance suitable for delivery of the polypeptides to the patient. Sterile water, alcohol, fats, waxes, and inert solids may be used as the carrier. Pharmaceutically- acceptable adjuvants, buffering agents, dispersing agents and the like, may also be incorporated into the pharmaceutical compositions. Such compositions can contain a single polypeptide or may contain two or more polypeptides.

It may be desirable to couple a polypeptide as hereinbefore described to immunoglobulins, e. g. IgG, or to lymphoid cells from the patient being treated in order to promote tolerance. Such an approach is described in Bradley-Mullen, Activation of Distinct Subsets of T Suppressor Cells with Type III Pneumococcal Polysaccharide Coupled to Syngeneic Spleen Cells, in: IMMUNOLOGICAL TOLERANCE TO SELF AND NON-SELF, Buttisto et al., eds., Annals N. Y. Acad. Sci. Vol. 392, pp 156-166,1982. Alternatively, the polypeptides may be modified to maintain or enhance binding to the MHC while reducing or eliminating binding to the associated T-cell receptor. In this way, the modified polypeptides may compete with natural tTG to inhibit helper T-cell activation and thus inhibit the immune response. In all cases, care should be taken that administration of the pharmaceutical compositions of the present invention ameliorate but do not potentiate the autoimmune response.

Pharmaceutical compositions as described above are useful for parenteral administration. Preferably, the compositions will be administered parenterally, i. e. subcutaneously, intramuscularly, or intravenously. Thus, compositions for parenteral administration to a patient comprise a solution or dispersion of the polypeptides in an acceptable carrier, as described above. The concentration of the polypeptides in the pharmaceutical composition can vary widely, i. e. from less than about 0.1% by weight, usually being at least about 1% by weight to as much as 20% by weight or more. Typical pharmaceutical compositions for intramuscular injection would be made up to contain, for example, 1 ml of sterile buffered water and 1 to 100 Ag of a purified polypeptide of the present invention. A typical composition for intravenous infusion could be made up to contain 100 to 500 ml of sterile Ringer's solution and 100 to 500 mg of a purified polypeptide of the present invention. Actual methods for preparing parenterally administrable compositions are well known in the art and described in more detail in various sources, including, for example, Remington's Pharmaceutical Science, 15th Edition, Mack Publishing Company, Easton, Pa. (1980).

In addition to using a polypeptide as disclosed and described herein directly in pharmaceutical compositions, it is also possible to use a polypeptide to enhance tolerance to tTG in a subject suspected of suffering from, susceptible to, having or recovering from autoimmune disease associated with an immune reaction to tTG, employing the following principles. More particularly, peripheral blood lymphocytes can be collected from the subject in a conventional manner and stimulated by exposure to a polypeptide as described above. Usually, other mitogens and growth enhancers will be present, e. g., phytohemagglutinin, interleukin 2, and the like. Proliferating T-helper cells may be isolated and cloned, also under the stimulation of a polypeptide according to the present invention. Clones which continue to proliferate may then be used to prepare therapeutic compositions for the subject. The cloned T-cells may be attenuated, e. g. by exposure to radiation, and administered to the subject in order to induce tolerance. Alternatively, the T-cell receptor or portions thereof may be isolated by conventional protein purification methods from the cloned T-cells and administered to the individual. Such immunotherapy methods are described generally in Sinha et al. (1990) Science 248: 1380-1388.

In some cases, after a T-helper cell has been cloned as described above, it may be possible to develop therapeutic peptides from the T-cell receptor, where the peptides would be beneficial for treating a patient population suspected of suffering from, susceptible to, having or recovering from autoimmune disease associated with an immune reaction to tTG. In such cases, the T-cell receptor gene may be isolated and cloned by conventional techniques and peptides based on the receptor produced by recombinant techniques as described above. The recombinantly-produced peptides may then be incorporated in pharmaceutical compositions as described above.

A method of cloning lymphocytes produced in response to tTG comprises:

providing a source of lymphocytes;

contacting the lymphocytes with a polypeptide according to the present invention substantially as hereinbefore described, so as to effect proliferation of said lymphocytes; and

isolating and cloning the proliferating lymphocytes.

Cloned lymphocytes prepared as above, may also be used in the therapeutic treatment of autoimmune disease associated with an immune reaction to tTG. A pharmaceutical composition comprises cloned lymphocytes prepared as above, together with a pharmaceutically acceptable carrier, diluent or excipient therefore. Such cloned lymphocytes may also be used in the manufacture of a medicament for the treatment of autoimmune disease associated with an immune reaction to tTG, in particular coeliac disease.

One or more therapeutic agents may be identified as providing a therapeutic effect by interaction with amino acids 1 to 89 of tTG, and/or amino acids 401 to 494 of tTG, or amino acids 401 to 491 of tTG. Such therapeutic agents may be used in therapeutically interacting with amino acids 1 to 89 of tTG, and/or amino acids 401 to 494 of tTG or amino acids 401 to 491 of Ttg and as such, may be used in the therapeutic treatment of an autoimmune disease associated with an immune reaction to tTG.

A method of treating autoimmune disease associated with an immune reaction to tTG in a subject comprises initially detecting autoantibodies or lymphocytes produced in response to tTG in a sample of body fluid obtained from the subject substantially as hereinbefore described, thereby providing a diagnosis of autoimmune disease in the subject, and administering to the subject a therapeutically effective amount of at least one therapeutic agent effective in the treatment of such autoimmune disease, such as a polypeptide substantially as hereinbefore described.

A method of treating autoimmune disease associated with an immune reaction to tTG in a subject (in particular a human subject) comprises administering to the subject a therapeutically effective amount of a therapeutic agent identified as providing a therapeutic effect by interaction with amino acids 1 to 89 of tTG, and/or amino acids 401 to 494 of tTG, or amino acids 401 to 491 of tTG.

The amount of therapeutic agent administered will depend on the specific autoimmune disease state being treated, possibly the age of the patient and will ultimately be at the discretion of an attendant physician.

The kit of the invention may be provided in combination together with a therapeutically effective amount of at least one therapeutic agent effective in the treatment of autoimmune disease associated with an immune reaction to tTG substantially as hereinbefore described.

Substantially as hereinbefore described, the sample of body fluid being screened by the present invention will typically comprise blood samples or other fluid blood fractions, such as in particular serum samples or plasma samples, but the sample may in principle be another biological fluid, such as saliva or urine or solubilised tissue extracts.

The present invention will now be illustrated with reference to the accompanying Figures and Examples, which do not limit the invention in any way. However, not all of these Figures and Examples are illustrative of the invention.

Figure 1 shows results of SDS PAGE (9%) analysis of purified recombinant human tTG for use in the method and kit according to the invention.

Figure 2 is a table showing binding of autoantibodies to tTG in sera from coeliac patients with full length and modified $^{35}$S-tTG proteins.

Figure 3 is a table showing monoclonal antibody (namely monoclonal antibodies A3-6B5 and A5-4E6) binding to full length and modified $^{35}$S-tTG proteins.

Figure 4 shows a schematic example of the method of the present invention when an antibody is employed.

Figure 5 is a graph showing binding (absorbance at 450nm in the vertical axis) of tTG with autoantibodies to tTG in different patients sera (horizontal axis). The binding is measured in the presence or absence of mouse tTG monoclonal antibody A3-6B5.

Figure 6 is a graph showing binding (absorbance at 450nm in the vertical axis) of tTG with autoantibodies to tTG in different patients sera (horizontal axis). The binding is measured in the presence or absence of mouse tTG monoclonal antibody A5-4E6.

Figure 7 depicts cDNA derived from human tTG, encoding amino acids 1 to 89 of human tTG.

Figure 8 depicts amino acids 1 to 89 of human tTG.

Figure 9 depicts cDNA derived from guinea pig tTG, encoding amino acids 1 to 89 of guinea pig tTG.

Figure 10 depicts amino acids 1 to 89 of guinea pig tTG.

Figure 11 depicts cDNA derived from bovine tTG, encoding amino acids 1 to 89 of bovine tTG.

Figure 12 depicts amino acids 1 to 89 of bovine tTG.

Figure 13 depicts cDNA derived from mouse tTG, encoding amino acids 1 to 89 of mouse tTG.

Figure 14 depicts amino acids 1 to 89 of mouse tTG.

Figure 15 depicts cDNA derived from rat tTG, encoding amino acids 1 to 89 of rat tTG.

Figure 16 depicts amino acids 1 to 89 of rat tTG.

Figure 17 depicts cDNA derived from human tTG, encoding amino acids 401 to 491 of human tTG.

Figure 18 depicts amino acids 401 to 491 of human tTG.

Figure 19 depicts cDNA derived from guinea pig tTG, encoding amino acids 401 to 494 of guinea pig tTG.

Figure 20 depicts amino acids 401 to 494 of guinea pig tTG.

Figure 21 depicts cDNA derived from bovine tTG, encoding amino acids 401 to 491 of bovine tTG.

Figure 22 depicts amino acids 401 to 491 of bovine tTG.

Figure 23 depicts cDNA derived from mouse tTG, encoding amino acids 401 to 491 of mouse tTG.

Figure 24 depicts amino acids 401 to 491 of mouse tTG.

Figure 25 depicts cDNA derived from rat tTG, encoding amino acids 401 to 491 of rat tTG.

Figure 26 depicts amino acids 401 to 491 of rat tTG.

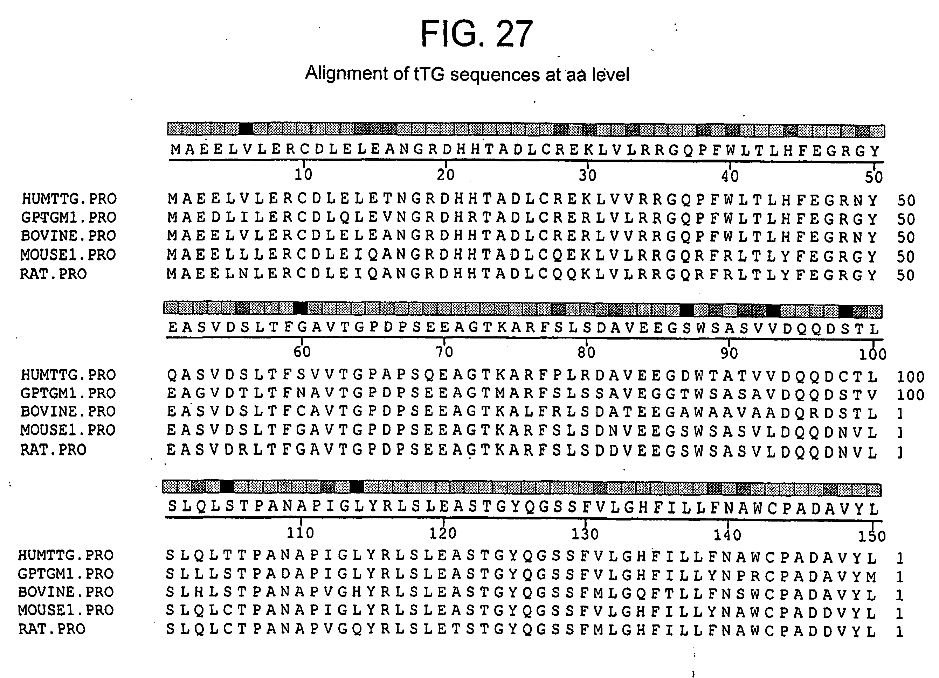

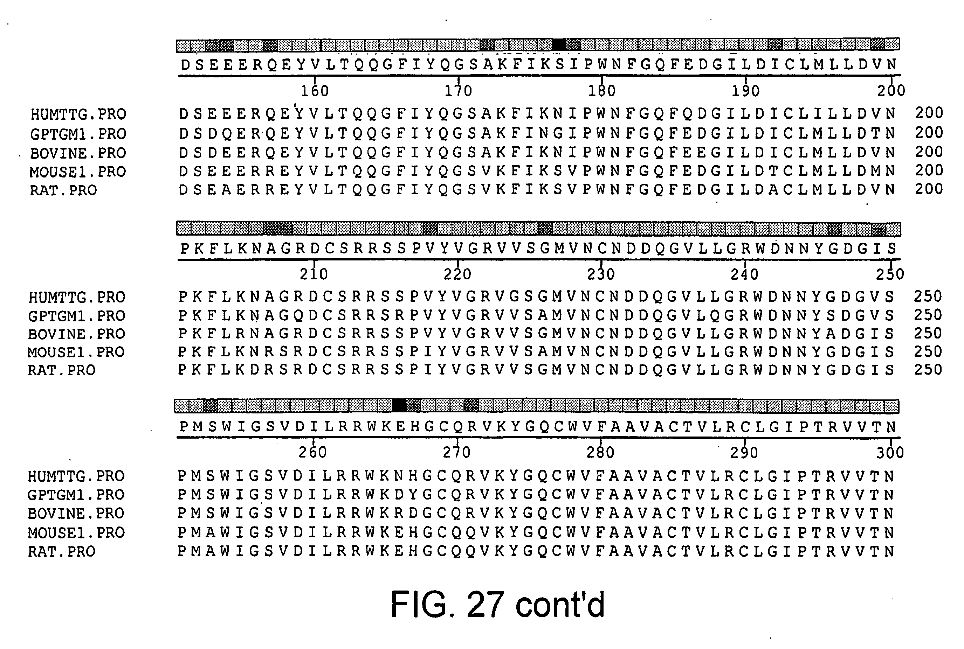

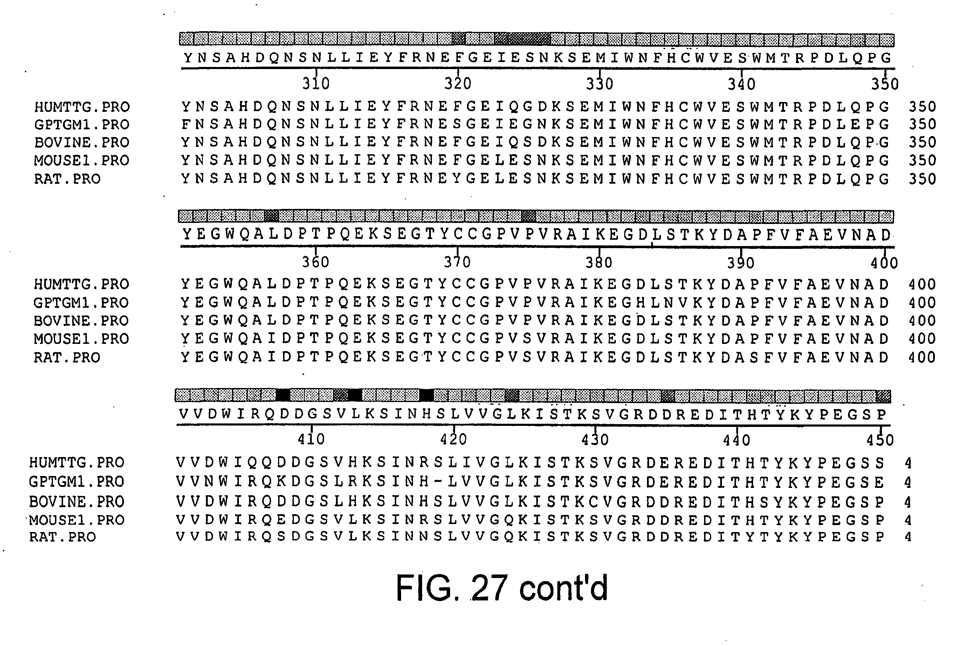

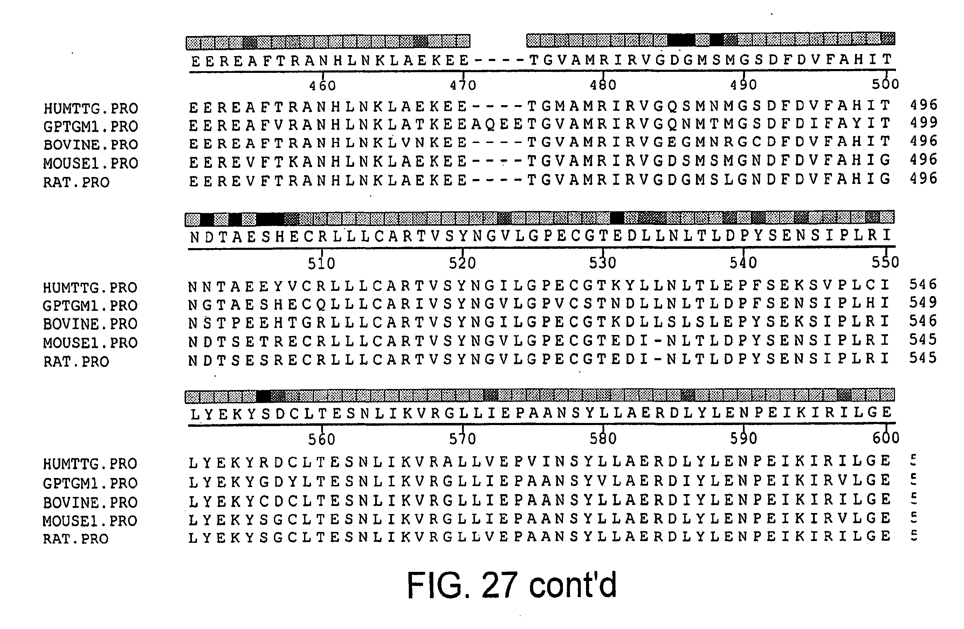

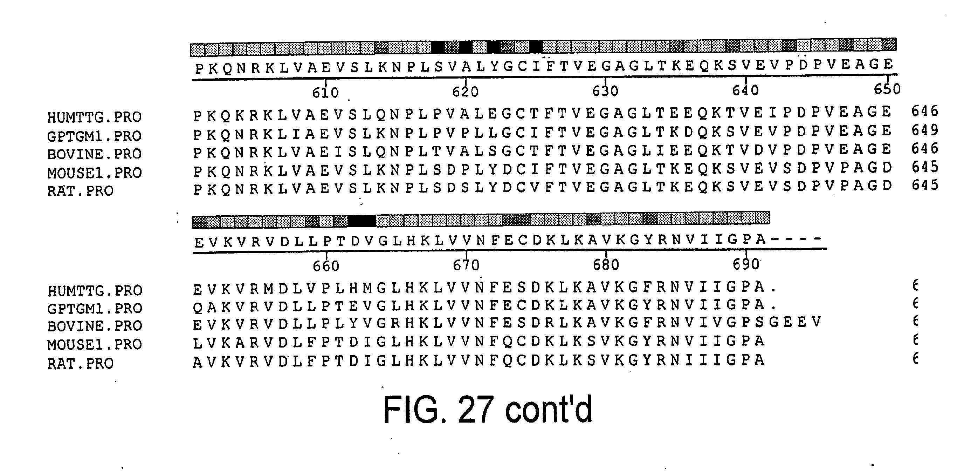

Figure 27 depicts the alignment of tTG sequences (human, guinea pig, bovine, mouse and rat) at the amino acid level.

## Examples

### Identification of regions of tTG which are recognized by tTG autoantibodies

Full length human tTG cDNA (endothelial cell isoform) was cloned into pTZ18R (Pharmacia) under control of the SP6 promoter. This construct was used to produce a reference full length tTG protein. Various in-frame deletions and truncations of the tTG gene were carried out using either naturally occurring restriction endonuclease sites or PCR mediated modifications to the sequence. The modified tTG constructs were cloned into pTZ18R (as above).

Full length and modified tTG proteins were expressed in an in vitro transcription/translation rabbit reticulocyte system (Promega) incorporating $^{35}$S-methionine (Amersham Pharmacia Biotech) and purified as described before (Colls J, et al. Clin. Chem. 1995; 41/3 :375-380). Table 1 shows the vectors used and the corresponding tTG amino acid residues expressed following the *in vitro* transcription/translation described above.

Table 1

| Vector Name | Expressed residues | Non-expressed (deleted) residues |
| --- | --- | --- |
| pTGM19 | 1-687 (full length) | None |
| pTGM25 | 1-628 | 629-687 |
| pTGM22 | 1-491 | 492-687 |
| pTGM24 | 1-400 | 401-687 |
| pTGM26 | 90-687 | 1-89 |
| pTGM27 | 172-687 | 1-171 |
| pTGM28 | 207-687 | 1-206 |
| pTGM29 | 281-687 | 1-280 |
| pTGM30 | 356-687 | 1-355 |
| pTGM31 | 399-687 | 1-398 |

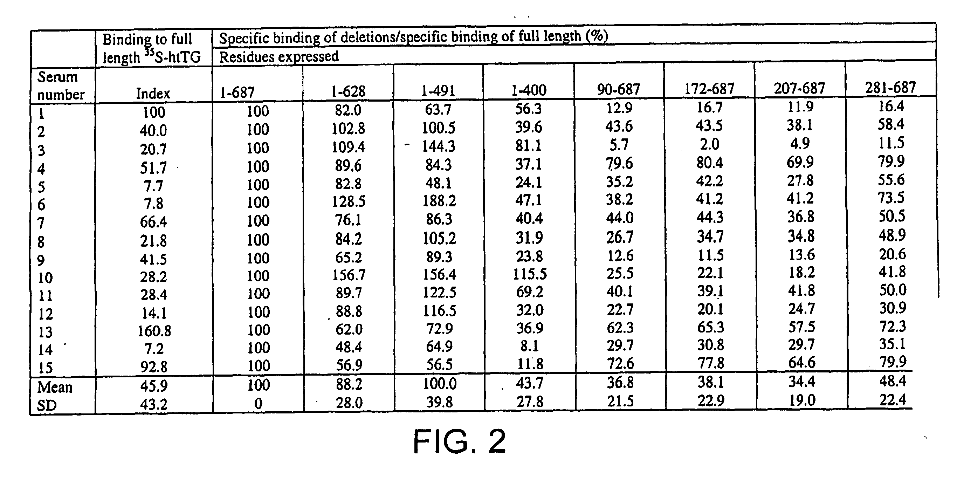

Binding of tTG autoantibodies to the purified full length and modified tTG proteins was analysed using an immunoprecipitation assay (Nakachi K, et al Clin Chim Acta, Volume 304, pages 75 to 84, 2002). Briefly, 10 μL of undiluted serum (in duplicate) was incubated with 50 μL of $^{35}$S-tTG (approx 15,000 dpm diluted in 10 mmol/L Tris HCl pH 7.6, 150mmol/L NaCl, 2 mmol/L EDTA, 0.1% Tween 20, 10g/L bovine serum albumin, 0.2g/L NaN$_3$; Assay Buffer) in Millipore filtration plates overnight at' 4˚C. Anti-human IgA agarose (50μl) was then added and the plates incubated for 4 hours at 4˚C. After incubation the reaction mixtures in the wells were washed 3 times. The wells were then punched out into vials containing 1mL of scintillation liquid and counted in a beta-counter. Figure 2 shows binding of tTG autoantibodies with different modified tTG proteins. The results are expressed as % binding relative to binding to the full length tTG reference protein.

### Serum samples

Serum from 34 patients with coeliac disease were used in the study. The diagnosis was based on typical clinical symptoms and was confirmed by positive duodenal biopsy. The sera tested positive for endomysial antibody and autoantibodies to tTG by standard ELISA. In addition sera from healthy blood donors (n=10) negative for autoantibodies to tTG were used.

Production of mouse monoclonal antibodies

Both guinea pig tTG (Sigma, Poole, UK) and recombinant human tTG (rhtTG) were used as antigens to immunise mice. Monoclonal antibodies (Mabs) were cloned using standard techniques. Selected tTG Mabs were grown in culture and purified by protein A affinity chromatography. The location of the binding sites of the mouse monoclonal antibodies was determined by an immunoprecipitation assay using the modified $^{35}$S-labelled tTG proteins as described above, except that anti-mouse IgG was added for precipitation. When required, purified IgG was labelled with $^{125}$I using the chloramine T method.

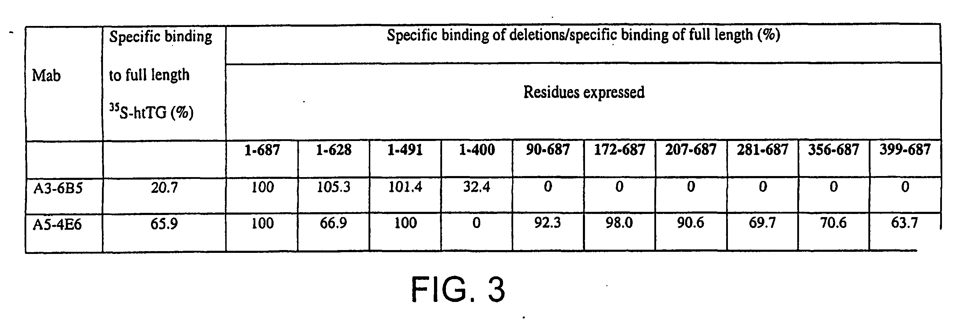

Analysis of the mouse Mabs by immunoprecipitation with modified $^{35}$S-labelled tTG proteins indicated that the epitope for A3-6B5 Mab was contained within aa 1-89 of the human tTG protein and the epitope for A5-4E6 Mab was contained within aa 401-491 of the human tTG protein (Figure 3).

Inhibition of autoantibodies to tTG binding with tTG by mouse tTG Mabs

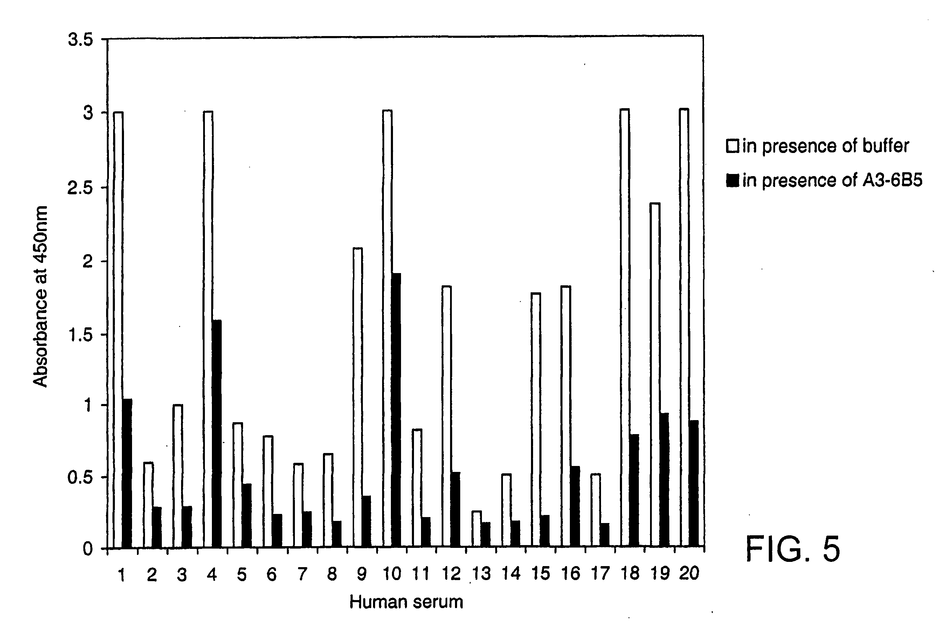

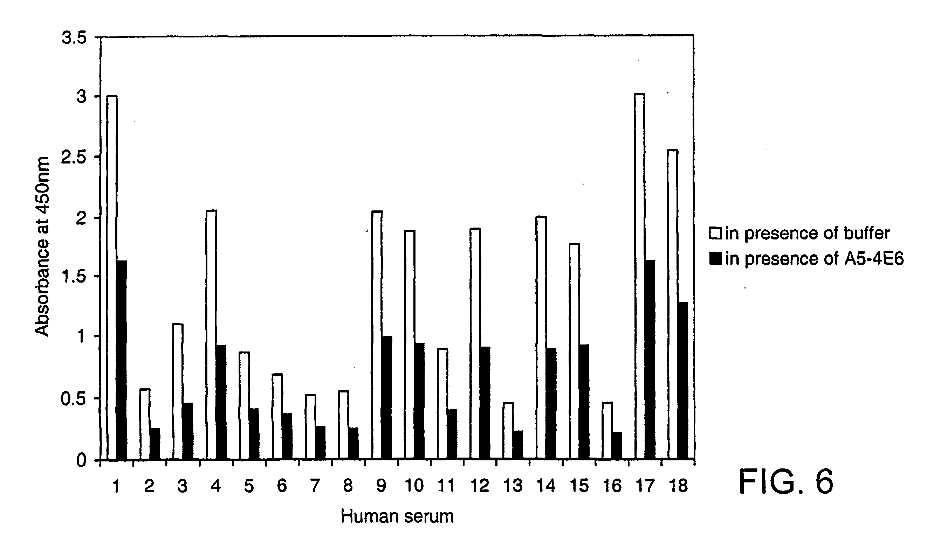

The inhibition of autoantibodies to tTG binding with tTG by mouse tTG Mabs was investigated in a modified version of an ELISA described in Nakachi K, et al. Clin Chim Acta (Volume 304, pages 75 to 84, 2002). To streptavidin coated plate wells 50/$\mu$L of tTG-biotin was added plus 10$\mu$L of Mab (200 $\mu$g/ml) or Assay Buffer and incubated for 30 mins. 40$\mu$L of patients' sera (typically diluted 1:100) was then added and incubated for 30 mins. After this step all wells were aspirated, washed four times and then 100$\mu$L of anti-human IgA-horseradish peroxidase (HRP) was added and incubated for 30 mins. After aspiration and four washes, 100$\mu$L of tetramethyl benzidine (TMB) solution was added and the plate was incubated for 15 mins in the dark. 50$\mu$L of stop solution was then added and absorbance read at 450nm in a plate reader. All steps were performed at room temperature, all incubations except the final TMB incubation were done with 200rpm shaking. tTG-biotin, Mabs and human sera were diluted in Assay Buffer. The results are shown in Figure 5 which shows binding of tTG with autoantibodies to tTG in different patients sera in the presence or absence of mouse tTG monoclonal antibody A3-6B5 and Figure 6 which shows binding of tTG with autoantibodies to tTG in different patients sera in the presence or absence of mouse tTG monoclonal antibody A5-4E6.

Preparation of purified recombinant human tTG

Recombinant human tTG was expressed in the yeast *Saccharomyces cerevisiae* strain c13ABYS86 using an expression system and culture techniques described previously (Powell M, et al. Clin Chim Acta 1996; 256: 175-188). Recombinant human tTG extracted from the yeast was partially purified by anion exchange chromatography and preparations of tTG of greater than 95% purity were obtained by affinity chromatography using mouse tTG Mab (A5-6C7) coupled to CNBr Sepharose. The purity of the purified tTG was assessed by SDS PAGE analysis on a 9% acrylamide gel followed by staining with Coomassie Blue as shown in Figure 1.

Assay for screening a sample of body, fluid for autoantibodies to tTG based on the inhibition of $^{125}$I-Mab binding to recombinant human tTG

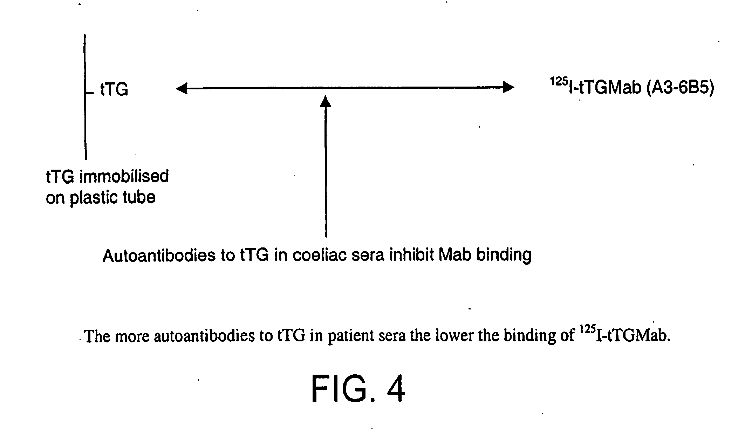

An assay was set up based on the principles of the schematic example shown in Figure 4. 4mL Nunc Maxisorp Startubes (Life Technologies) were coated overnight at 4°C with 250$\mu$L of 10$\mu$g/mL recombinant human tTG (rhtTG). Following aspiration and three washes with 500$\mu$L Assay Buffer, tubes were incubated for 30 minutes at room temperature with 500$\mu$L of Post-Coat Buffer containing bovine serum albumin. After two washes with Assay Buffer, tubes were incubated with 100$\mu$L of serum sample diluted 1:5 in Assay Buffer for 60 minutes at room temperature on a shaker. Following aspiration and three washes with 500$\mu$L of Assay Buffer, tubes were incubated on a shaker for 30 minutes at room temperature with 100$\mu$L of $^{125}$I-labelled tTG Mab A3-6B5 (30,000 cpm; diluted in Assay Buffer). After a final aspiration and 3 washes with 500$\mu$L of Assay Buffer, tubes were counted for 1 minute in a gamma counter.

The results, measured in counts per minute (cpm), were used to calculate the % inhibition of $^{125}$I-Mab binding as follows:

$$\texttt{\%inhibition = 100-} \left[ \frac{\underline{A} \times 100}{B} \right]$$

wherein,

A = $^{125}$I-Mab (cpm) bound to rhtTG in the presence of test sera as a % of total cpm of material added to the tube; and

B = $^{125}$I-Mab (cpm) bound to rhtTG in the presence of healthy blood donor sera (mean of 2 or 3 sera) as a % of total cpm of material added to the tube.

Results and Discussion

Figure 2 showed that C-terminal deletions of amino acids (aa) 492-687 of the tTG protein had no or little effect on tTG autoantibody binding. More extensive C-terminal deletion (aa 401-687) resulted in lower tTG autoantibody binding in 14/15 sera studied. N-terminal deletion of aa 1-89 of the tTG protein resulted in lower tTG autoantibody binding in all sera studied.

Therefore, the studies with modified tTG proteins described herein showed that tTG autoantibody binding sites on tTG are heterogenous and are dependent on the aa sequences in the N-terminal and central part of the molecule. Furthermore, these studies showed that aa 1-89, and aa 401-491 of human tTG protein are important for binding of tTG autoantibodies present in the coeliac sera studied. (aa 401-491 of human tTG correspond to aa 401-494 of tTG in other species, such as guinea pig tTG.)

Analysis of mouse Mabs by immunoprecipitation with modified $^{35}$S-labelled tTG proteins indicated that the epitope for A3-6B5 Mab was contained within aa 1-89 of the human tTG protein and the epitope for A5-4E6 Mab was contained within aa 401-491 of the human tTG protein (Figure 3). Furthermore, these Mabs, namely A3-6B5 Mab and A5-4E6 Mab, inhibited binding of tTG autoantibody positive sera with tTG in ELISA (see Figures 5 and 6).

Identification of amino acid sequences of the tTG molecule important for binding of autoantibodies to tTG tested, and production of mouse Mabs reactive with these amino acid sequences, allowed the development of a new assay to screen for the presence of autoantibodies to tTG in a sample of body fluid as described above.

The results of the assay based on autoantibodies to tTG in test sera inhibiting $^{125}$I-Mab A3-6B5 binding with recombinant human tTG are shown in Table 2.

Table 2 Results of assay based on the inhibition of $^{125}$I-Mab binding to recombinant human tTG

| Serum Number | % Inhibition of $^{125}$I- A3-6B5 Mab binding | tTG Autoantibodies ELISA u/mL | $^{35}$S rhtTG Autoantibodies IPA Index |
|---|---|---|---|
| Coeliac | | | |
| 1 | 79 | 1000 | 100 |
| 2 | 35 | 110 | 52 |
| 3 | 91 | 42 | 7.7 |
| 4 | 49 | 150 | 21 |
| 5 | 89 | 1000 | 44 |
| 6 | 88 | 51 | 107 |
| 7 | 89 | 1000 | 122 |
| 8 | 42 | 102 | 107 |
| 9 | 48 | 45 | 3.3 |
| | | | |
| Healthy blood donors | | | |
| 1 | -11 | Neg | Neg |
| 2 | 13 | Neg | Neg |
| 3 | -1 | Neg | Neg |
| 4 | 3 | Neg | Neg |
| 5 | -3 | Neg | Neg |

Nine sera from coeliac patients which tested positive for tTG autoantibodies by immunoprecipitation assay (tTG autoantibodies index from 3.3-107) and ELISA (42-1000 u/mL) were analysed. All these sera inhibited binding of $^{125}$I-Mab to rhtTG coated on the tube (35 to 91% inhibition).

In contrast, sera negative for autoantibodies to tTG only had a small effect on the binding of $^{125}$I-Mab to rhtTG (-11 to 13% inhibition).

## Claims

1. A method of screening for autoantibodies to tTG in a sample of body fluid obtained from a subject suspected of suffering from, susceptible to, having or recovering from autoimmune disease associated with an immune reaction to tTG, said method comprising:

   (a) contacting said sample with

      (i) either a polypeptide which consists of amino acid numbers 1 to 89 of tTG, or full length tTG, and
      (ii) at least one competitor antibody or antibody fragment which binds to amino acid numbers 1 to 89 of tTG, and is capable of competing with autoantibodies to tTG in the interaction thereof with a polypeptide or amino acid sequence which consists of amino acid numbers 1 to 89 of tTG,

   under conditions that allow interaction of tTG with autoantibodies to tTG, so as to permit either said polypeptide or said full length tTG, depending on which is used according to (i), to interact with either autoantibodies to tTG present in said sample, or said competitor antibody or antibody fragment; and
   (b) monitoring the interaction of either said polypeptide or said full length tTG with said autoantibodies present in said sample, thereby providing an indication of the presence of said autoantibodies to tTG in said sample.

2. A method according to claim 1, which employs a polypeptide that consists of amino acid numbers 1 to 89 of tTG.

3. A method according to claim 1, which employs full length tTG.

4. A method according to any of claims 1 to 3, wherein said competitor antibody or antibody fragment is a monoclonal antibody.

5. A method according to any of claims 1 to 4, wherein said monitoring comprises comparing the difference in the level of interaction of said competitor antibody or antibody fragment in the presence of said sample, wherein a reduction in the level of interaction of said competitor is indicative of the presence of autoantibodies to tTG in said sample.

6. A kit for screening for autoantibodies to tTG in a sample of body fluid obtained from a subject suspected of suffering from, susceptible to, having or recovering from autoimmune disease associated with an immune reaction to tTG, said kit comprising:

   (a) either a polypeptide which consists of amino acid numbers 1 to 89 of tTG, or full length tTG;
   (b) at least one competitor antibody or antibody fragment which binds to amino acid numbers 1 to 89 of tTG, and is capable of competing with autoantibodies to tTG in the interaction thereof with a polypeptide or amino acid sequence which consists of amino acid numbers 1 to 89 of tTG;
   (c) means for contacting (i) said sample of body fluid from said subject, (ii) either said polypeptide or said full length tTG, depending on which is used according to (a), and (iii) said competitor antibody or antibody fragment under conditions that allow interaction of tTG wth autoantibodies to tTG, so as to permit said polypeptide, or full length tTG, to interact with either autoantibodies to tTG present in said sample, or said competitor; and
   (d) means for monitoring the interaction of either said polypeptide or said full length tTG with said autoantibodies present in said sample, thereby providing an indication of the presence of said autoantibodies to tTG in said sample.

7. A kit according to claim 6, which includes a polypeptide that consists of amino acid numbers 1 to 89 of tTG.

8. A kit according to claim 6, which includes full length tTG.

9. A kit according to any of claims 6 to 8, wherein said competitor antibody or antibody fragment is a monoclonal antibody.

**10.** A method of screening according to any one of claims 1 to 5, or a kit according to any one of claims 6 to 9, wherein the competitor antibody or antibody fragment is a monoclonal antibody or an active fragment thereof.

**Patentansprüche**

**1.** Screeningverfahren für Autoantikörper gegen tTG in einer Körperflüssigkeitsprobe, erhalten von einem Probanden, der im Verdacht steht, an einer Autoimmunerkrankung, die mit einer Immunreaktion gegen tTG zusammenhängt, zu leiden, dafür anfällig ist, sie hat oder sich von ihr erholt, wobei das Verfahren umfasst:

(a) In-Kontakt-Bringen der Probe mit

(i) entweder einem Polypeptid, das aus den Aminosäuren Nummer 1 bis 89 von tTG besteht, oder tTG voller Länge und
(ii) mindestens einem Kompetitor-Antikörper oder -Antikörperfragment, der bzw. das an die Aminosäuren Nummer 1 bis 89 von tTG bindet und fähig ist, mit Autoantikörpern gegen tTG bei deren Wechselwirkung mit einem Polypeptid oder einer Aminosäuresequenz, die aus den Aminosäuren Nummer 1 bis 89 des tTG besteht, zu kompetieren,

unter Bedingungen, die eine Wechselwirkung von tTG mit Autoantikörpern gegen tTG erlauben, um so zu ermöglichen, dass entweder das Polypeptid oder das tTG voller Länge, je nachdem welches gemäß (i) verwendet wird, entweder mit in der Probe anwesenden Autoantikörpern gegen tTG oder mit dem Kompetitor-Antikörper oder -Antikörperfragment in Wechselwirkung tritt; und
(b) Überwachen der Wechselwirkung entweder des Polypeptids oder des tTG voller Länge mit den in der Probe anwesenden Autoantikörpern, um **dadurch** einen Hinweis auf die Anwesenheit der Autoantikörper gegen tTG in der Probe bereitzustellen.

**2.** Verfahren gemäß Anspruch 1, welches ein Polypeptid einsetzt, das aus den Aminosäuren Nummer 1 bis 89 von tTG besteht.

**3.** Verfahren gemäß Anspruch 1, welches tTG voller Länge einsetzt.

**4.** Verfahren gemäß einem der Ansprüche 1 bis 3, wobei es sich bei dem Kompetitor-Antikörper oder -Antikörperfragment um einen monoklonalen Antikörper handelt.

**5.** Verfahren gemäß einem der Ansprüche 1 bis 4, wobei das Überwachen das Vergleichen des Unterschieds im Ausmaß der Wechselwirkung des Kompetitor-Antikörpers oder -Antikörperfragments in Anwesenheit der Probe umfasst, wobei eine Senkung des Ausmaßes der Wechselwirkung des Kompetitors auf die Anwesenheit von Autoantikörpern gegen tTG in der Probe hinweist.

**6.** Kit zum Screening für Autoantikörper gegen tTG in einer Körperflüssigkeitsprobe, erhalten von einem Probanden, der im Verdacht steht, an einer Autoimmunerkrankung, die mit einer Immunreaktion gegen tTG zusammenhängt, zu leiden, dafür anfällig ist, sie hat oder sich von ihr erholt, wobei der Kit umfasst:

(a) entweder ein Polypeptid, das aus den Aminosäuren Nummer 1 bis 89 von tTG besteht, oder tTG voller Länge;
(b) mindestens einen Kompetitor-Antikörper oder ein -Antikörperfragment, der bzw. das an die Aminosäuren Nummer 1 bis 89 von tTG bindet und fähig ist, mit Autoantikörpern gegen tTG bei deren Wechselwirkung mit einem Polypeptid oder einer Aminosäuresequenz, die aus den Aminosäuren Nummer 1 bis 89 des tTG besteht, zu kompetieren;
(c) Mittel zum In-Kontakt-Bringen (i) der Körperflüssigkeitsprobe des Probanden, (ii) entweder des Polypeptids oder des tTG voller Länge, je nachdem welches gemäß (a) verwendet wird, und (iii) des Kompetitor-Antikörpers oder -Antikörperfragments unter Bedingungen, welche eine Wechselwirkung von tTG mit Autoantikörpern gegen tTG erlauben, um so zu ermöglichen, dass das Polypeptid oder tTG voller Länge entweder mit in der Probe anwesenden Autoantikörpern gegen tTG oder mit dem Kompetitor in Wechselwirkung tritt; und
(d) Mittel zum Überwachen der Wechselwirkung entweder des Polypeptids oder des tTG voller Länge mit den in der Probe anwesenden Autoantikörpern, um **dadurch** einen Hinweis auf die Anwesenheit der Autoantikörper gegen tTG in der Probe bereitzustellen.

**7.** Kit gemäß Anspruch 6, der ein Polypeptid einschließt, das aus den Aminosäuren Nummer 1 bis 89 von tTG besteht.

**8.** Kit gemäß Anspruch 6, der tTG voller Länge einschließt.

**9.** Kit gemäß einem der Ansprüche 6 bis 8, wobei es sich bei dem Kompetitor-Antikörper oder -Antikörperfragment um einen monoklonalen Antikörper handelt.

**10.** Screeningverfahren gemäß einem der Ansprüche 1 bis 5 oder Kit gemäß einem der Ansprüche 6 bis 9, wobei es sich bei dem Kompetitor-Antikörper oder -Antikörperfragment um einen monoklonalen Antikörper oder ein aktives Fragment davon handelt.

**Revendications**

**1.** Procédé de criblage d'auto-anticorps contre la tTG dans un échantillon de fluide corporel obtenu à partir d'un sujet supposé atteint de, sensible à, ayant ou se rétablissant d'une maladie auto-immune associée à une réaction immunitaire envers la tTG, ledit procédé consistant à :

(a) mettre en contact ledit échantillon avec

(i) un polypeptide qui consiste en acides aminés numéro 1 à 89 de tTG, ou de tTG de longueur totale, et
(ii) au moins un anticorps compétiteur ou un fragment d'anticorps qui se lie aux acides aminés numéro 1 à 89 de tTG, et qui est capable de concurrencer les auto-anticorps contre tTG lors de leur interaction avec un polypeptide ou une séquence d'acides aminés qui consiste en acides aminés de numéro 1 à 89 de tTG,

dans des conditions qui permettent l'interaction de tTG avec les anticorps contre tTG, de manière à permettre audit polypeptide ou audit tTG de longueur totale, en fonction de ce qui est utilisé selon (i), d'interagir avec des auto-anticorps contre tTG présents dans ledit échantillon, ou ledit anticorps compétitif ou un fragment d'anticorps ; et
(b) surveiller l'interaction dudit polypeptide ou dudit tTG de longueur totale avec lesdits auto-anticorps présents dans ledit échantillon, en fournissant ainsi une indication de la présence desdits auto-anticorps contre tTG dans ledit échantillon.

**2.** Procédé selon la revendication 1, qui utilise un polypeptide qui consiste en acides aminés de numéro 1 à 89 de tTG.

**3.** Procédé selon la revendication 1, qui utilise la tTG de longueur totale.

**4.** Procédé selon l'une quelconque des revendications 1 à 3, dans lequel ledit anticorps compétiteur ou fragment d'anticorps est un anticorps monoclonal.

**5.** Procédé selon l'une quelconque des revendications 1 à 4, dans lequel ladite surveillance consiste à comparer la différence de niveau d'interaction dudit anticorps compétiteur ou fragment d'anticorps en présence dudit échantillon, où une réduction du niveau d'interaction dudit compétiteur indique la présence d'auto-anticorps contre tTG dans ledit échantillon.

**6.** Kit de criblage d'anticorps contre tTG dans un échantillon de fluide corporel obtenu à partir d'un sujet supposé atteint de, sensible à, ayant ou se rétablissant d'une maladie auto-immune associée à une réaction immunitaire envers la tTG, ledit kit comprenant :

(a) un polypeptide qui consiste en acides aminés numéro 1 à 89 de tTG, ou de tTG de longueur totale ;
(b) au moins un anticorps compétiteur ou fragment d'anticorps qui se lie aux acides aminés numéros 1 à 89 de tTG, et qui est capable de concurrencer les auto-anticorps contre tTG lors de leur interaction avec un polypeptide ou une séquence d'acides aminés qui consiste en acides aminés numéro 1 à 89 de tTG ;
(c) des moyens pour mettre en contact (i) ledit échantillon de fluide corporel provenant dudit sujet, (ii) ledit polypeptide ou ladite tTG de longueur totale, en fonction de ce qui est utilisé selon (a), et (iii) ledit anticorps compétiteur ou fragment d'anticorps dans des conditions qui permettent l'interaction de tTG avec des anticorps contre tTG, de manière à permettre audit polypeptide, ou tTG de longueur totale, d'interagir avec les auto-anticorps contre tTG présents dans ledit échantillon, ou ledit compétiteur ; et

(d) des moyens pour surveiller l'interaction dudit polypeptide ou de ladite tTG de longueur totale avec lesdits auto-anticorps présents dans ledit échantillon, en fournissant ainsi une indication de la présence desdits auto-auto-auto-auto-anticorps contre tTG dans ledit échantillon.

7. Kit selon la revendication 6, qui comprend un polypeptide qui consiste en acides aminés numéro 1 à 89 de tTG.

8. Kit selon la revendication 6, qui comprend la tTG de longueur totale.

9. Kit selon l'une quelconque des revendications 6 à 8, où ledit anticorps compétiteur ou fragment d'anticorps est un anticorps monoclonal.

10. Procédé de criblage selon l'une quelconque des revendications 1 à 5, ou kit selon l'une quelconque des revendications 6 à 9, où l'anticorps compétiteur ou fragment d'anticorps est un anticorps monoclonal ou un fragment actif de celui-ci.

FIG. 1

| Serum number | Binding to full length $^{35}$S-htTG | Specific binding of deletions/specific binding of full length (%) | | | | | | | |
|---|---|---|---|---|---|---|---|---|---|
| | Index | Residues expressed | | | | | | | |
| | | 1-687 | 1-628 | 1-491 | 1-400 | 90-687 | 172-687 | 207-687 | 281-687 |
| 1 | 100 | 100 | 82.0 | 63.7 | 56.3 | 12.9 | 16.7 | 11.9 | 16.4 |
| 2 | 40.0 | 100 | 102.8 | 100.5 | 39.6 | 43.6 | 43.5 | 38.1 | 58.4 |
| 3 | 20.7 | 100 | 109.4 | 144.3 | 81.1 | 5.7 | 2.0 | 4.9 | 11.5 |
| 4 | 51.7 | 100 | 89.6 | 84.3 | 37.1 | 79.6 | 80.4 | 69.9 | 79.9 |
| 5 | 7.7 | 100 | 82.8 | 48.1 | 24.1 | 35.2 | 42.2 | 27.8 | 55.6 |
| 6 | 7.8 | 100 | 128.5 | 188.2 | 47.1 | 38.2 | 41.2 | 41.2 | 73.5 |
| 7 | 66.4 | 100 | 76.1 | 86.3 | 40.4 | 44.0 | 44.3 | 36.8 | 50.5 |
| 8 | 21.8 | 100 | 84.2 | 105.2 | 31.9 | 26.7 | 34.7 | 34.8 | 48.9 |
| 9 | 41.5 | 100 | 65.2 | 89.3 | 23.8 | 12.6 | 11.5 | 13.6 | 20.6 |
| 10 | 28.2 | 100 | 156.7 | 156.4 | 115.5 | 25.5 | 22.1 | 18.2 | 41.8 |
| 11 | 28.4 | 100 | 89.7 | 122.5 | 69.2 | 40.1 | 39.1 | 41.8 | 50.0 |
| 12 | 14.1 | 100 | 88.8 | 116.5 | 32.0 | 22.7 | 20.1 | 24.7 | 30.9 |
| 13 | 160.8 | 100 | 62.0 | 72.9 | 36.9 | 62.3 | 65.3 | 57.5 | 72.3 |
| 14 | 7.2 | 100 | 48.4 | 64.9 | 8.1 | 29.7 | 30.8 | 29.7 | 35.1 |
| 15 | 92.8 | 100 | 56.9 | 56.5 | 11.8 | 72.6 | 77.8 | 64.6 | 79.9 |
| Mean | 45.9 | 100 | 88.2 | 100.0 | 43.7 | 36.8 | 38.1 | 34.4 | 48.4 |
| SD | 43.2 | 0 | 28.0 | 39.8 | 27.8 | 21.5 | 22.9 | 19.0 | 22.4 |

## FIG. 2

| Mab | Specific binding to full length $^{35}S$-htTG (%) | Specific binding of deletions/specific binding of full length (%) | | | | | | | | | |
|---|---|---|---|---|---|---|---|---|---|---|---|
| | | Residues expressed | | | | | | | | | |
| | | 1-687 | 1-628 | 1-491 | 1-400 | 90-687 | 172-687 | 207-687 | 281-687 | 356-687 | 399-687 |
| A3-6B5 | 20.7 | 100 | 105.3 | 101.4 | 32.4 | 0 | 0 | 0 | 0 | 0 | 0 |
| A5-4E6 | 65.9 | 100 | 66.9 | 100 | 0 | 92.3 | 98.0 | 90.6 | 69.7 | 70.6 | 63.7 |

# FIG. 3

EP 1 360 280 B1

tTG ⟵⟶ $^{125}$I-tTGMab (A3-6B5)

tTG immobilised
on plastic tube

Autoantibodies to tTG in coeliac sera inhibit Mab binding

The more autoantibodies to tTG in patient sera the lower the binding of $^{125}$I-tTGMab.

# FIG. 4

FIG. 5

EP 1 360 280 B1

FIG. 6

EP 1 360 280 B1

```
        10          20          30          40
 ⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊
 atggccgaggagctggtcttagagaggtgtgatctggagc 40
 tggagaccaatggccgagaccaccacacggccgacctgtg 80
 ccgggagaagctggtggtgcgacggggccagcccttctgg 120
 ctgaccctgcactttgagggccgcaactaccaggccagtg 160
 tagacagtctcaccttcagtgtcgtgaccggcccagcccc 200
        210         220         230         240
 ⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊
 tagccaggaggccgggaccaaggcccgtttttccactaaga 240
 gatgctgtggaggagggtgactggaca 267
```

# FIG. 7

Human tTG
DNA sequence that encodes amino acids 1-89

```
        10          20          30          40
 ⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊
 MAEELVLERCDLELETNGRDHHTADLCREKLVVRRGQPFW 40
 LTLHFEGRNYQASVDSLTFSVVTGPAPSQEAGTKARFPLR 80
 DAVEEGDWT 89
```

# FIG. 8

Human tTG
Protein sequence of amino acids 1-89

31

EP 1 360 280 B1

```
        10          20          30          40
  atggcagaggatctgatcctggagagatgtgatttgcagc 40
  tggaggtcaatggccgcgaccaccgcacggccgacctgtg 80
  ccgggagaggctggtgttgcggcggggccagcccttctgg 120
  ctgacgctgcactttgagggccgtggctacgaggctggtg 160
  tggacactctcaccttcaacgctgtgaccggcccagatcc 200

        210         220         230         240
  cagtgaggaggccgggactatggcccggttctcactgtcc 240
  agtgctgtcgaggggggcacctggtca 267
```

# FIG. 9

Guinea Pig tTG
DNA sequence that encodes amino acids 1-89

```
        10          20          30          40
  MAEDLILERCDLQLEVNGRDHRTADLCRERLVLRRGQPFW 40
  LTLHFEGRGYEAGVDTLTFNAVTGPDPSEEAGTMARFSLS 80
  SAVEGGTWS 89
```

# FIG. 10

Guinea Pig tTG
Protein sequence of amino acids 1-89

32

```
          10        20        30        40
 ┴┴┴┴┴┴┴┴┴┴┴┴┴┴┴┴┴┴┴┴┴┴┴┴┴┴┴┴┴┴┴┴┴┴┴┴┴┴┴┴┴
atggccgaggagctggtcctggagagatgtgacctggagc 40
tggaggccaatggccgtgaccaccacacagctgacctgtg 80
cagggaaaggctggtagtgcggcggggccagcccttctgg 120
ctgactctgcactttgagggccggaactatgaggccagcg 160
tggacagcctcaccttttgtgctgtgactggcccagaccc 200
          210       220       230       240
 ┴┴┴┴┴┴┴┴┴┴┴┴┴┴┴┴┴┴┴┴┴┴┴┴┴┴┴┴┴┴┴┴┴┴┴┴┴┴┴┴┴
cagtgaggaggccgggaccaaggccctcttcaggctgtcc 240
gatgctacggaggaggggggcctgggca 267
```

# FIG. 11

Bovine tTG
DNA sequence encoding amino acids 1-89

```
          10        20        30        40
 ┴┴┴┴┴┴┴┴┴┴┴┴┴┴┴┴┴┴┴┴┴┴┴┴┴┴┴┴┴┴┴┴┴┴┴┴┴┴┴┴┴
MAEELVLERCDLELEANGRDHHTADLCRERLVVRRGQPFW 40
LTLHFEGRNYEASVDSLTFCAVTGPDPSEEAGTKALFRLS 80
DATEEGAWA 89
```

# FIG. 12

Bovine tTG
Protein sequence of amino acids 1-89

```
         10        20        30        40
 ⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐
atggcagaggagctgctcctggagaggtgtgatttggaga 40
ttcaggccaatggccgtgaccaccacacggccgacctatg 80
ccaagagaaactggtgctgcgtcgtggtcagcgcttccgg 120
ctgactctgtacttcgagggccgtggctacgaggccagcg 160
tggacagcctcacgttcggtgctgtgaccggcccagatcc 200
        210       220       230       240
 ⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐
cagtgaagaggcagggaccaaggcccgcttctcactgtct 240
gacaatgtggaggagggatcttggtca 267
```

# FIG. 13

Mouse tTG
DNA sequence encoding residues 1-89

```
         10        20        30        40
 ⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐
MAEELLLERCDLEIQANGRDHHTADLCQEKLVLRRGQRFR 40
LTLYFEGRGYEASVDSLTFGAVTGPDPSEEAGTKARFSLS 80
DNVEEGSWS 89
```

# FIG. 14

Mouse tTG
Protein sequence of amino acids 1-89

```
        10          20          30          40
  ⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊
  atggcagaggagctgaacctggagaggtgcgacttggaga   40
  tacaggccaatggccgtgatcaccacacggccgacctgtg   80
  ccaacagaaactggtgctgcggcgaggccagcgcttccgg  120
  ctgacactgtacttcgagggccgtggctatgaggccagcg  160
  tggacagacttacatttggtgccgtgaccggcccagatcc  200
       210         220         230         240
  ⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊
  cagtgaagaggcagggaccaaggcccgattctcactgtct  240
  gacgatgtggaggagggatcctggtca  267
```

# FIG. 15

Rat tTG
DNA sequence encoding residues 1-89

```
        10          20          30          40
  ⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊⌊
  MAEELNLERCDLEIQANGRDHHTADLCQQKLVLRRGQRFR   40
  LTLYFEGRGYEASVDRLTFGAVTGPDPSEEAGTKARFSLS   80
  DDVEEGSWS  89
```

# FIG. 16

Rat tTG
Protein sequence of amino acids 1-89

```
        10          20          30          40
 ⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐
gtggtagactggatccagcaggacgatgggtctgtgcaca  40
aatccatcaaccgttccctgatcgttgggctgaagatcag  80
cactaagagcgtgggccgagacgagcgggaggatatcacc  120
cacacctacaaatacccagaggggtcctcagaggagaggg  160
aggccttcacaagggcgaaccacctgaacaaactggccga  200
        210         220         230         240
 ⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐
gaaggaggagacagggatggccatgcggatccgtgtgggc  240
cagagcatgaacatgggcagtgactttgacgtc  273
```

# FIG. 17

Human tTG
DNA sequence encoding residues 401-491

```
        10          20          30          40
 ⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐⌐
VVDWIQQDDGSVHKSINRSLIVGLKISTKSVGRDEREDIT  40
HTYKYPEGSSEEREAFTRANHLNKLAEKEETGMAMRIRVG  80
QSMNMGSDFDV  91
```

# FIG. 18

Human tTG
Protein sequence of residues 401-491

```
         10        20        30        40
  ┴┴┴┴┴┴┴┴┴┴┴┴┴┴┴┴┴┴┴┴┴┴┴┴┴┴┴┴┴┴┴┴┴┴┴┴┴┴┴
  gtggtgaactggatccggcagaaagatgggtccctgcgca 40
  agtccatcaaccatttggttgtggggctgaagatcagtac 80
  taagagtgtgggccgcgatgagcgagaggacatcacccac 120
  acctacaagtacccagagggatctgaagaggagcgggaag 160
  cttttgttagggccaaccacctaaataaactggccacaaa 200
         210       220       230       240
  ┴┴┴┴┴┴┴┴┴┴┴┴┴┴┴┴┴┴┴┴┴┴┴┴┴┴┴┴┴┴┴┴┴┴┴┴┴┴┴
  ggaagaggctcaggaggaaacgggagtggccatgcggatc 240
  cgtgtgggccagaacatgactatgggcagtgactttgaca 280
  tc 282
```

# FIG. 19

Guinea Pig tTG
DNA sequence encoding amino acids 401-494
(equivalent to 401-491 in human sequence)

```
         10        20        30        40
  ┴┴┴┴┴┴┴┴┴┴┴┴┴┴┴┴┴┴┴┴┴┴┴┴┴┴┴┴┴┴┴┴┴┴┴┴┴┴┴
  VVNWIRQKDGSLRKSINHLVVGLKISTKSVGRDEREDITH 40
  TYKYPEGSEEEREAFVRANHLNKLATKEEAQEETGVAMRI 80
  RVGQNMTMGSDFDI 94
```

# FIG. 20

Guinea Pig tTG
Protein sequence of amino acids 401-494

```
          10        20        30        40
  .....|....|....|....|....|....|....|....|
  gtggtggactggatccggcaggacgatgggtctctgcaca  40
  aatccatcaaccactccctggtggtggggctgaagatcag  80
  cacaaagtgtgtgggcagagatgatcgggaggacatcacc  120
  cacagctacaagtacccggaggggtccccagaggaaaggg  160
  aagccttcacaagagccaaccatctgaacaaactggttaa  200
          210       220       230       240
  .....|....|....|....|....|....|....|....|
  caaagaggagacaggggtggccatgcggatccgtgtgggc  240
  gagggcatgaacagaggctgcgacttcgacgtc  273
```

# FIG. 21

Bovine tTG
DNA sequence that encodes amino acids 401-491

```
          10        20        30        40
  ....|....|....|....|....|....|....|....|
  VVDWIRQDDGSLHKSINHSLVVGLKISTKCVGRDDREDIT  40
  HSYKYPEGSPEEREAFTRANHLNKLVNKEETGVAMRIRVG  80
  EGMNRGCDFDV  91
```

# FIG. 22

Bovine tTG
Protein sequence of amino acids 401-491

38

```
        10          20          30          40
  ｜｜｜｜｜｜｜｜｜｜｜｜｜｜｜｜｜｜｜｜｜｜｜｜｜｜｜｜｜｜｜｜｜｜｜｜｜｜｜｜
  gtggtggactggatccggcaggaagatgggtctgtgctca 40
  aatccatcaaccgttccttggtcgtggggcagaagatcag 80
  cactaagagtgtgggccgtgatgaccgggaggacatcacc 120
  catacctacaagtacccagaggggtcacccgaggagaggg 160
  aagtcttcaccaaggccaaccacctgaacaaactggcaga 200
        210         220         230         240
  ｜｜｜｜｜｜｜｜｜｜｜｜｜｜｜｜｜｜｜｜｜｜｜｜｜｜｜｜｜｜｜｜｜｜｜｜｜｜｜｜
  gaaagaggagacaggggtggccatgcgcatccgagtgggg 240
  gacagtatgagcatgggcaacgacttcgacgtg 273
```

# FIG. 23

Mouse tTG
DNA sequence that encodes amino acids 401-491

```
        10          20          30          40
  ｜｜｜｜｜｜｜｜｜｜｜｜｜｜｜｜｜｜｜｜｜｜｜｜｜｜｜｜｜｜｜｜｜｜｜｜｜｜｜｜
  VVDWIRQEDGSVLKSINRSLVVGQKISTKSVGRDDREDIT 40
  HTYKYPEGSPEEREVFTKANHLNKLAEKEETGVAMRIRVG 80
  DSMSMGNDFDV 91
```

# FIG. 24

Mouse tTG
Protein sequence of amino acids 401-491

39

```
              10         20         30         40
    ┴┴┴┴┴┴┴┴┴┴┴┴┴┴┴┴┴┴┴┴┴┴┴┴┴┴┴┴┴┴┴┴┴┴┴┴┴┴┴┴┴┴┴┴
    gtggtggactggatccggcagtcagatgggtctgtgctca 40
    aatccatcaacaattccctggtcgtggggcagaaaatcag 80
    cactaagagcgtgggccgtgatgaccgggaggacatcacc 120
    tatacctacaagtacccagaggggtccccagaggaaaggg 160
    aagtcttcaccagagccaaccacctgaacaaactggcaga 200
              210        220        230        240
    ┴┴┴┴┴┴┴┴┴┴┴┴┴┴┴┴┴┴┴┴┴┴┴┴┴┴┴┴┴┴┴┴┴┴┴┴┴┴┴┴┴┴┴┴
    gaaagaggagacaggggtggccatgcggatccgagtgggg 240
    gatggtatgagcttgggcaatgactttgacgtg 273
```

# FIG. 25

Rat tTG
DNA sequence that encodes amino acids 401-491

```
              10         20         30         40
    ┴┴┴┴┴┴┴┴┴┴┴┴┴┴┴┴┴┴┴┴┴┴┴┴┴┴┴┴┴┴┴┴┴┴┴┴┴┴┴┴┴┴┴┴
    VVDWIRQSDGSVLKSINNSLVVGQKISTKSVGRDDREDIT 40
    YTYKYPEGSPEEREVFTRANHLNKLAEKEETGVAMRIRVG 80
    DGMSLGNDFDV 91
```

# FIG. 26

Rat tTG
Protein sequence of amino acids 401-491

# FIG. 27

## Alignment of tTG sequences at aa level

```
MAEELVLERCDLELEANGRDHHTADLCREKLVLRRGQPFWLTLHFEGRGY
         10        20        30        40        50
```

| | | |
|---|---|---|
| HUMTTG.PRO | MAEELVLERCDLELETNGRDHHTADLCREKLVVRRGQPFWLTLHFEGRNY | 50 |
| GPTGM1.PRO | MAEDLILERCDLQLEVNGRDHRTADLCRERLVLRRGQPFWLTLHFEGRGY | 50 |
| BOVINE.PRO | MAEELVLERCDLELEANGRDHHTADLCRERLVVRRGQPFWLTLHFEGRNY | 50 |
| MOUSE1.PRO | MAEELLLERCDLEIQANGRDHHTADLCQEKLVLRRGQRFRLTLYFEGRGY | 50 |
| RAT.PRO | MAEELNLERCDLEIQANGRDHHTADLCQQKLVLRRGQRFRLTLYFEGRGY | 50 |

```
EASVDSLTFGAVTGPDPSEEAGTKARFSLSDAVEEGSWSASVVDQQDSTL
         60        70        80        90       100
```

| | | |
|---|---|---|
| HUMTTG.PRO | QASVDSLTFSVVTGPAPSQEAGTKARFPLRDAVEEGDWTATVVDQQDCTL | 100 |
| GPTGM1.PRO | EAGVDTLTFNAVTGPDPSEEAGTMARFSLSSAVEGGTWSASAVDQQDSTV | 100 |
| BOVINE.PRO | EASVDSLTFCAVTGPDPSEEAGTKALFRLSDATEEGAWAAVAADQRDSTL | 1 |
| MOUSE1.PRO | EASVDSLTFGAVTGPDPSEEAGTKARFSLSDNVEEGSWSASVLDQQDNVL | 1 |
| RAT.PRO | EASVDRLTFGAVTGPDPSEEAGTKARFSLSDDVEEGSWSASVLDQQDNVL | 1 |

```
SLQLSTPANAPIGLYRLSLEASTGYQGSSFVLGHFILLFNAWCPADAVYL
        110       120       130       140       150
```

| | | |
|---|---|---|
| HUMTTG.PRO | SLQLTTPANAPIGLYRLSLEASTGYQGSSFVLGHFILLFNAWCPADAVYL | 1 |
| GPTGM1.PRO | SLLLSTPADAPIGLYRLSLEASTGYQGSSFVLGHFILLYNPRCPADAVYM | 1 |
| BOVINE.PRO | SLHLSTPANAPVGHYRLSLEASTGYQGSSFMLGQFTLLFNSWCPADAVYL | 1 |
| MOUSE1.PRO | SLQLCTPANAPIGLYRLSLEASTGYQGSSFVLGHFILLYNAWCPADDVYL | 1 |
| RAT.PRO | SLQLCTPANAPVGQYRLSLETSTGYQGSSFMLGHFILLFNAWCPADDVYL | 1 |

EP 1 360 280 B1

EP 1 360 280 B1

```
              D S E E R Q E Y V L T Q Q G F I Y Q G S A K F I K S I P W N F G Q F E D G I L D I C L M L L D V N
                          160            170            180            190            200
```

|            |                                                                                                          |     |
|------------|----------------------------------------------------------------------------------------------------------|-----|
| HUMTTG.PRO | D S E E R Q E Y V L T Q Q G F I Y Q G S A K F I K N I P W N F G Q F Q D G I L D I C L I L L D V N          | 200 |
| GPTGM1.PRO | D S D Q E R Q E Y V L T Q Q G F I Y Q G S A K F I N G I P W N F G Q F E D G I L D I C L M L L D T N        | 200 |
| BOVINE.PRO | D S D E E R Q E Y V L T Q Q G F I Y Q G S A K F I K N I P W N F G Q F E E G I L D I C L M L L D V N        | 200 |
| MOUSE1.PRO | D S E E E R R E Y V L T Q Q G F I Y Q G S V K F I K S V P W N F G Q F E D G I L D T C L M L L D M N        | 200 |
| RAT.PRO    | D S E A E R R E Y V L T Q Q G F I Y Q G S V K F I K S V P W N F G Q F E D G I L D A C L M L L D V N        | 200 |

```
              P K F L K N A G R D C S R R S S P V Y V G R V V S G M V N C N D D Q G V L L G R W D N N Y G D G I S
                          210            220            230            240            250
```

|            |                                                                                                          |     |
|------------|----------------------------------------------------------------------------------------------------------|-----|
| HUMTTG.PRO | P K F L K N A G R D C S R R S S P V Y V G R V G S G M V N C N D D Q G V L L G R W D N N Y G D G V S        | 250 |
| GPTGM1.PRO | P K F L K N A G Q D C S R R S R P V Y V G R V V S A M V N C N D D Q G V L Q G R W D N N Y S D G V S        | 250 |
| BOVINE.PRO | P K F L R N A G R D C S R R S S P V Y V G R V V S G M V N C N D D Q G V L L G R W D N N Y A D G I S        | 250 |
| MOUSE1.PRO | P K F L K N R S R D C S R R S S P I Y V G R V V S A M V N C N D D Q G V L L G R W D N N Y G D G I S        | 250 |
| RAT.PRO    | P K F L K D R S R D C S R R S S P I Y V G R V V S G M V N C N D D Q G V L L G R W D N N Y G D G I S        | 250 |

```
              P M S W I G S V D I L R R W K E H G C Q R V K Y G Q C W V F A A V A C T V L R C L G I P T R V V T N
                          260            270            280            290            300
```

|            |                                                                                                          |
|------------|----------------------------------------------------------------------------------------------------------|
| HUMTTG.PRO | P M S W I G S V D I L R R W K N H G C Q R V K Y G Q C W V F A A V A C T V L R C L G I P T R V V T N        |
| GPTGM1.PRO | P M S W I G S V D I L R R W K D Y G C Q R V K Y G Q C W V F A A V A C T V L R C L G I P T R V V T N        |
| BOVINE.PRO | P M S W I G S V D I L R R W K R D G C Q R V K Y G Q C W V F A A V A C T V L R C L G I P T R V V T N        |
| MOUSE1.PRO | P M A W I G S V D I L R R W K E H G C Q Q V K Y G Q C W V F A A V A C T V L R C L G I P T R V V T N        |
| RAT.PRO    | P M A W I G S V D I L R R W K E H G C Q Q V K Y G Q C W V F A A V A C T V L R C L G I P T R V V T N        |

# FIG. 27 cont'd

```
            YNSAHDQNSNLLIEYFRNEFGEIESNKSEMIWNFHCWVESWMTRPDLQPG
                  310       320       330       340       350
HUMTTG.PRO  YNSAHDQNSNLLIEYFRNEFGEIQGDKSEMIWNFHCWVESWMTRPDLQPG  350
GPTGM1.PRO  FNSAHDQNSNLLIEYFRNESGEIEGNKSEMIWNFHCWVESWMTRPDLEPG  350
BOVINE.PRO  YNSAHDQNSNLLIEYFRNEFGEIQSDKSEMIWNFHCWVESWMTRPDLQPG  350
MOUSE1.PRO  YNSAHDQNSNLLIEYFRNEFGELESNKSEMIWNFHCWVESWMTRPDLQPG  350
RAT.PRO     YNSAHDQNSNLLIEYFRNEYGELESNKSEMIWNFHCWVESWMTRPDLQPG  350


            YEGWQALDPTPQEKSEGTYCCGPVPVRAIKEGDLSTKYDAPFVFAEVNAD
                  360       370       380       390       400
HUMTTG.PRO  YEGWQALDPTPQEKSEGTYCCGPVPVRAIKEGDLSTKYDAPFVFAEVNAD  400
GPTGM1.PRO  YEGWQALDPTPQEKSEGTYCCGPVPVRAIKEGHLNVKYDAPFVFAEVNAD  400
BOVINE.PRO  YEGWQALDPTPQEKSEGTYCCGPVPVRAIKEGDLSTKYDAPFVFAEVNAD  400
MOUSE1.PRO  YEGWQAIDPTPQEKSEGTYCCGPVSVRAIKEGDLSTKYDAPFVFAEVNAD  400
RAT.PRO     YEGWQAIDPTPQEKSEGTYCCGPVSVRAIKEGDLSTKYDASFVFAEVNAD  400


            VVDWIRQDDGSVLKSINHSLVVGLKISTKSVGRDDREDITHTYKYPEGSP
                  410       420       430       440       450
HUMTTG.PRO  VVDWIQQDDGSVHKSINRSLIVGLKISTKSVGRDEREDITHTYKYPEGSS  4
GPTGM1.PRO  VVNWIRQKDGSLRKSINH-LVVGLKISTKSVGRDEREDITHTYKYPEGSE  4
BOVINE.PRO  VVDWIRQDDGSLHKSINHSLVVGLKISTKCVGRDDREDITHSYKYPEGSP  4
MOUSE1.PRO  VVDWIRQEDGSVLKSINRSLVVGQKISTKSVGRDDREDITHTYKYPEGSP  4
RAT.PRO     VVDWIRQSDGSVLKSINNSLVVGQKISTKSVGRDDREDITYTYKYPEGSP  4
```

# FIG. 27 cont'd

EP 1 360 280 B1

EEREAFTRANHLNKLAEKEE----TGVAMRIRVGDGMSMGSDFDVFAHIT
460      470      480      490      500

HUMTTG.PRO    EEREAFTRANHLNKLAEKEE----TGMAMRIRVGQSMNMGSDFDVFAHIT   496
GPTGM1.PRO    EEREAFVRANHLNKLATKEEAQEETGVAMRIRVGQNMTMGSDFDIFAYIT   499
BOVINE.PRO    EEREAFTRANHLNKLVNKEE----TGVAMRIRVGEGMNRGCDFDVFAHIT   496
MOUSE1.PRO    EEREVFTKANHLNKLAEKEE----TGVAMRIRVGDSMSMGNDFDVFAHIG   496
RAT.PRO       EEREVFTRANHLNKLAEKEE----TGVAMRIRVGDGMSLGNDFDVFAHIG   496

NDTAESHECRLLLCARTVSYNGVLGPECGTEDLLNTLDPYSENSIPLRI
510      520      530      540      550

HUMTTG.PRO    NNTAEEYVCRLLLCARTVSYNGILGPECGTKYLLNTLEPFSEKSVPLCI   546
GPTGM1.PRO    NGTAESHECQLLLCARIVSYNGVLGPVCSTNDLLNTLDPFSENSIPLHI   549
BOVINE.PRO    NSTPEEHTGRLLLCARTVSYNGILGPECGTKDLLSLSLEPYSEKSIPLRI   546
MOUSE1.PRO    NDTSETRECRLLLCARTVSYNGVLGPECGTEDI-NLTLDPYSENSIPLRI   545
RAT.PRO       NDTSESRECRLLLCARTVSYNGVLGPECGTEDI-NLTLDPYSENSIPLRI   545

LYEKYSDCLTESNLIKVRGLLIEPAANSYLLAERDLYLENPEIKIRILGE
560      570      580      590      600

HUMTTG.PRO    LYEKYRDCLTESNLIKVRALLVEPVINSYLLAERDLYLENPEIKIRILGE   5
GPTGM1.PRO    LYEKYGDYLTESNLIKVRGLLIEPAANSYVLAERDIYLENPEIKIRVLGE   5
BOVINE.PRO    LYEKYCDCLTESNLIKVRGLLIEPAANSYLLAERDIYLENPEIKIRILGE   5
MOUSE1.PRO    LYEKYSGCLTESNLIKVRGLLIEPAANSYLLAERDLYLENPEIKIRVLGE   5
RAT.PRO       LYEKYSGCLTESNLIKVRGLLVEPAANSYLLAERDLYLENPEIKIRILGE   5

# FIG. 27 cont'd

EP 1 360 280 B1

P K Q N R K L V A E V S L K N P L S V A L Y G C I F T V E G A G L T K E Q K S V E V P D P V E A G E

610        620        630        640        650

| | |
|---|---|
| HUMTTG.PRO | P K Q K R K L V A E V S L Q N P L P V A L E G C T F T V E G A G L T E E Q K T V E I P D P V E A G E    646 |
| GPTGM1.PRO | P K Q N R K L I A E V S L K N P L P V P L L G C I F T V E G A G L T K D Q K S V E V P D P V E A G E    649 |
| BOVINE.PRO | P K Q N R K L V A E I S L Q N P L T V A L S G C T F T V E G A G L I E E Q K T V D V P D P V E A G E    646 |
| MOUSE1.PRO | P K Q N R K L V A E V S L K N P L S D P L Y D C I F T V E G A G L T K E Q K S V E V S D P V P A G D    645 |
| RAT.PRO | P K Q N R K L V A E V S L K N P L S D S L Y D C V F T V E G A G L T K E Q K S V E V S D P V P A G D    645 |

E V K V R V D L L P T D V G L H K L V V N F E C D K L K A V K G Y R N V I I G P A - - - -

660        670        680        690

| | |
|---|---|
| HUMTTG.PRO | E V K V R M D L V P L H M G L H K L V V N F E S D K L K A V K G F R N V I I G P A .    6 |
| GPTGM1.PRO | Q A K V R V D L L P T E V G L H K L V V N F E C D K L K A V K G Y R N V I I G P A .    6 |
| BOVINE.PRO | E V K V R V D L L P L Y V G R H K L V V N F E S D R L K A V K G F R N V I V G P S G E E V    6 |
| MOUSE1.PRO | L V K A R V D L F P T D I G L H K L V V N F Q C D K L K S V K G Y R N V I I G P A    6 |
| RAT.PRO | A V K V R V D L F P T D I G L H K L V V N F Q C D K L K S V K G Y R N I I I G P A    6 |

# FIG. 27 cont'd

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9803872 A


**Non-patent literature cited in the description**

- Activation of Distinct Subsets of T Suppressor Cells with Type III Pneumococcal Polysaccharide Coupled to Syngeneic Spleen Cells. **Bradley-Mullen et al.** IMMUNOLOGICAL TOLERANCE TO SELF AND NON-SELF. Annals N. Y. Acad. Sci, 1982, vol. 392, 156-166
- Remington's Pharmaceutical Science. Mack Publishing Company, 1980
- **Sinha et al.** *Science,* 1990, vol. 248, 1380-1388
- **Colls J et al.** *Clin. Chem.,* 1995, vol. 41 (3), 375-380
- **Nakachi K et al.** *Clin Chim Acta,* 2002, vol. 304, 75-84
- **Powell M et al.** *Clin Chim Acta,* 1996, vol. 256, 175-188